# EUROPEAN PATENT SPECIFICATION

(11) **EP 4 062 179 B1**
(45) Date of publication and mention of the grant of the patent: **25.09.2024**
(21) Application number: 20833981.2
(22) Date of filing: 30.10.2020
(51) Int. Cl.: G01N 33/70

(54) **FLUORIMETRIC ANALYTICAL METHOD FOR THE DETERMINATION OF CREATININE IN CLINICALLY SIGNIFICANT BIOLOGICAL SAMPLES AND A FLUORIMETRIC REAGENT FOR USE IN THIS METHOD**
FLUORIMETRISCHES ANALYTISCHES VERFAHREN ZUR BESTIMMUNG VON KREATININ IN KLINISCH BEDEUTSAMEN BIOLOGISCHEN PROBEN UND EIN FLUORIMETRISCHES REAGENZ ZUR VERWENDUNG IN DIESEM VERFAHREN
PROCÉDÉ ANALYTIQUE FLUOROMÉTRIQUE POUR LA DÉTERMINATION DE LA CRÉATININE DANS DES ÉCHANTILLONS BIOLOGIQUES CLINIQUEMENT SIGNIFICATIFS ET RÉACTIF FLUOROMÉTRIQUE DESTINÉ À ÊTRE UTILISÉ DANS CE PROCÉDÉ

(30) Priority: 30.10.2019 PL 43166019
(43) Date of publication of application: 28.09.2022
(73) Proprietor: Uniwersytet Warszawski, 00-927 Warszawa (PL)
(72) Inventor: LEWINSKA, Izabela, 02-087 Warszawa (PL); MICHALEC, Michal, 26-612 Radom (PL); KONCKI, Robert, 02-930 Warszawa (PL); TYMECKI, Lukasz, 02-384 Warszawa (PL)
(86) International application number: PCT/PL2020/000083
(87) International publication number: WO 2021/086209

(56) References cited:
- EP-A1- 0 066 552
- US-A1- 2019 219 589
- BLASS K G ED - LOCKWOOD CHRISTINA M ET AL: "REACTIVITY OF CREATININE WITH ALKALINE 3,5-DINITROBENZOATE: A NEW FLUORESCENT KIDNEY FUNCTION TEST", CLINICAL BIOCHEMISTRY, ELSEVIER INC, US, CA, vol. 28, no. 2, 1 January 1995 (1995-01-01), pages 107 - 111, XP002070749, ISSN: 0009-9120, DOI: 10.1016/0009-9120(95)00004-S
- RAYMOND A. HELLER ET AL: "Kinetics of the reaction of p -dinitrobenzene with basic hydrogen peroxide", CANADIAN JOURNAL OF CHEMISTRY, vol. 65, no. 2, 1 February 1987 (1987-02-01), CA, pages 251 - 255, XP055770689, ISSN: 0008-4042, DOI: 10.1139/v87-041
- LEWINSKA IZABELA ET AL: "From the bottom of an old jar: A fluorometric method for the determination of creatinine in human serum", ANALYTICA CHIMICA ACTA, ELSEVIER, AMSTERDAM, NL, vol. 1135, 2 September 2020 (2020-09-02), pages 116 - 122, XP086297990, ISSN: 0003-2670, [retrieved on 20200902], DOI: 10.1016/J.ACA.2020.08.017

## Description

The present invention relates to a fluorometric method for the determination of creatinine in clinically significant samples and a reagent for use in the fluorimetric method for the determination of creatinine in clinically significant samples.

Creatinine is one of the metabolites of nitrogen compounds, present in all body fluids. It has no biological function and is removed from the bloodstream in kidneys (by glomerular filtration) and then excreted in urine from the body. Serum creatinine level is a marker of kidney filtration function and therefore a marker of a number of diseases.

In clinical laboratories, the accuracy and speed of a single creatinine determination in a clinically significant sample play an important role, and creatinine itself, beside glucose, is one of the most frequently determined metabolites. Creatinine level is routinely measured in serum and urine samples, mainly to assess kidney function, but also in diagnostics of diseases resulting in muscle loss, as an indication of urine dilution, and to predict the risk of acute renal failure.

There are many known methods of creatinine determination in biological samples. They cover the entire spectrum of instrumental techniques available today: from spectroscopic techniques (photometry, fluorimetry), through electrochemical techniques, including current and potentiometric methods, to separation techniques: chromatography and electromigration.

In analytical practice, the Jaffé method developed in 1886 [M. Jaffe, Z. Physiol. Chem. 10 (1886) 391-400] is recommended for routine creatinine determination in clinically significant samples, despite its numerous disadvantages, including-Low selectivity for among others glucose and proteins. In the case of determinations in more advanced investigations, definitely more selective enzymatic methods are used although they are also more expensive.

The Jaffé method for the determination of creatinine in biological samples is based on the reaction between creatinine and picric acid (2,4,6-trinitrophenol) in an alkaline environment. The reaction product is an orange creatinine-picric acid complex/adduct, which can be determined spectrophotometrically in the wavelength range 470-550 nm. One of the postulated course of the reaction may be presented in the following reaction scheme:

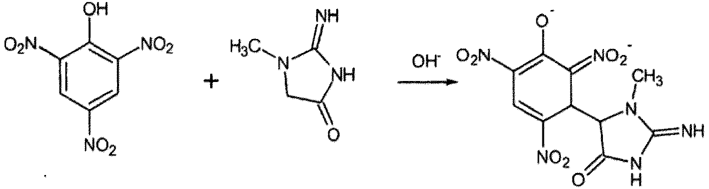

The Jaffé reaction was adapted to the colorimetric determination of creatinine in urine in 1904 [O. Folin and J. L. Morris, J. Biol. Chem. 17 (1914) 469-473] and in deproteinised blood in 1919 [O. Folin and H. Wu, J. Biol. Chem. 38 (1919) 81-110]. Typical reagent concentrations and analytical parameters for the Jaffé protocol are shown in Table 1.

**Table 1. Typical reagent concentrations and analytical parameters in the Jaffé protocol. [elaboration based on the technical manual for the Roche Cobas 5000 apparatus, CREJ2 assay protocol]**

| **Parameter** | **Value** |
|---|---|
| Concentration of picric acid | 38 mmol·L⁻¹ |
| Concentration of potassium base | 900 mmol·L⁻¹ |
| Analytical wavelength | 505-529 nm |
| Reference wavelength | 505 nm |
| Limit of creatinine quantification* | 15 µmol·L⁻¹ |
| Creatinine detection limit in serum** | 15 µmol·L⁻¹ |
| Creatinine detection limit in urine ** | 375 µmol·L⁻¹ |
| Linear range of the method | 15-55000 µmol·L⁻¹ |

| | |
|---|---|
| ** Quantification limit determined according to the requirements of EP17-A CLSI (Clinical and Laboratory Standards Institute) as the 95th percentile of values from n> 60 blank measurements in several independent series.* ** *The lower detection limit is the lowest measurable concentration of the analyte that can be distinguished from zero (3 standard deviations from the lowest standard, repeatability n> 21).* | |

One of the most significant drawbacks and disadvantages of the Jaffé method is the fact that the course of the reaction is highly temperature dependent, and therefore the slope of the orange complex calibration curve is also temperature dependent. In the wavelength range of 475-520 nm, the absorbance of the creatinine-picric acid complex/adduct increases with increasing temperature, with the degree of increase in absorbance being different for different wavelengths at which it is measured. For this reason, an increase in temperature by 1°C can cause an error in the measured creatinine concentration by up to 0.7 µmol·L⁻¹ if the measurement is made at 490 nm, while when the absorbance is measured at wavelengths longer than 500 nm, an error caused by temperature fluctuations is several times smaller. In addition, picric acid used in the Jaffé method, as a dry solid reagent, is an explosive substance and toxic to humans in case of contact with skin, inhalation and ingestion. Therefore, the preparation of the reagent must be carried out by trained personnel.

The main disadvantage of the Jaffé method, however, is its low selectivity. Creatinine is not the only compound that forms a red complex when reacted with picric acid, and a similar effect is also observed in the presence of acetone or glucose. The list of interferents of the Jaffé method includes more and more substances, including: proteins, bilirubin, glucose, ascorbic acid, acetoacetates and antibiotics belonging to the cephalosporin group, and the total number of known interferents already reaches over 200. Some of them increase and other reduce the signal, while in comparison with more reliable methods, the Jaffé method increases the obtained results by about 27 µmol·L⁻¹, at average [B. Wuyts et al., Clin. Chem. 49 (2003) 1011-1014].

Many attempts have been made to increase selectivity of the Jaffé method, but the only method routinely used in clinical analysis is a discriminant - sometimes called kinetic or two-point, measurement [D. L. Fabiny, Clin. Chem. 17 (1971) 696-700]. As the analytical signal, instead of the absorbance, the difference in absorbance measured at 80^{th} and 20^{th} second of the reaction at a wavelength of 520 nm is used. In this way, the influence of compounds, for example bilirubin, that react with picric acid with kinetics different to that of creatinine, is eliminated. Another advantage of using the two-point measurement is elimination of the influence of the sample color on the obtained result, because both blood serum and urine absorb radiation in the analytical range of the Jaffé method. The presented modification of the Jaffé method does not release it from all the known drawbacks. First of all, the influence of proteins on the measured creatinine concentration is not eliminated. Moreover, the double absorbance measurement creates a difficulty in the measurement methodology and causes the propagation of measurement uncertainty.

As an alternative to the attempts to modify the Jaffé method in order to improve its selectivity, it was proposed to replace picric acid with another nitro derivative of benzene, namely 3,5-dinitrobenzoic acid (DNBA) [A. Bollinger, Med. J. Aust. 2 (1936) 818-821; S. R. Benedict and J. A. Bahre, W. Langley and M. Evans, J. Biol. Chem. 114 (1936) 515-532; J. Biol. Chem. 115 (1936) 333-341]. The reaction is analogous to the reaction with picric acid according to the scheme:

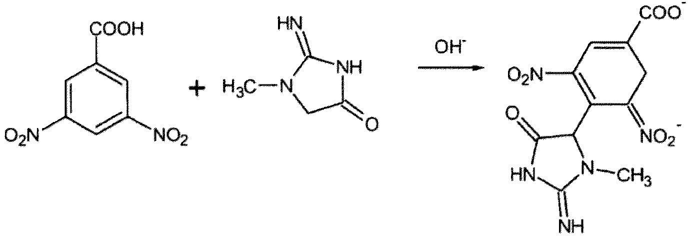

Due to the introduced change of the reagent, the method is more selective for creatinine than the classic Jaffé method, because compounds such as glucose, fructose, creatine or guanidine do not affect the measurement [J. Carr et al., Anal. Chem. 25 (1953) 1859-1863]. The kinetics of the creatinine reaction with 3,5-dinitrobenzoic acid is largely dependent on the concentration of sodium hydroxide: at low concentrations the product forms slowly but is stable over time, while at higher concentrations the reaction kinetics are faster but the product decomposes. The results with the best reliability are obtained with a 3,5-dinitrobenzoic acid solution in a concentration of 0.25 mmol·L⁻¹ and a sodium base solution of the same concentration, whereas the absorbance reading at 500 nm wavelength is made 60 minutes after mixing the reagents [J. Carr et al., Anal. Chem. 25 (1953) 1859-1863].

There are also reports on methods based on fluorimetric detection. The basis of the described fluorescent kidney function test is the creatinine reaction with an alkali metal salt of 3,5-dinitrobenzoic acid [K. G. Blass, Clin. Biochem. 28 (1995) 107-111; K. G. Blass, US 5,527,708 (1996)].

There is known a fluorimetric method of creatinine detection that employs a palladium (II) complex of naphthalimide derivative, which complex does not show fluorescence in a buffer of pH 7.2. After addition of creatinine solution, the palladium ion is removed from the complex and is bound by the analyte molecule. The ligand itself (naphthalimide derivative), devoid of the palladium ion, fluoresces with an emission maximum at a wavelength of 530 nm, when excited at a wavelength of 440 nm. The dependence of the creatinine concentration on the fluorescence intensity can be approximated by the square function within the range of creatinine concentrations ranging from 0 to 300 µmol·L⁻¹ [S. Pal et al., Chem. Commun. 52 (2016) 13706-13709]. The developed protocol shows a correlation with the results obtained by the Jaffé method.

There is known a fluorimetric method of creatinine determination involving its reaction with a chalcone derivative [S. Ellairaja et al., ACS Sensors. 3 (2018) 2463-2466]. This compound is characterised by a quantum yield of 0.85 at pH of 10 and, when excited by radiation of 407 nm wavelength, it has two emission maxima: one at a wavelength of 473 nm and the other at 587 nm, and the presence of creatinine shifts the emission maximum of the compound towards shorter waves. Other metabolites present in biological samples, such as urea, bilirubin, amino acids and proteins, and metal ions do not cause such a shift. The method is selective for creatinine, but is characterised by low precision (RSD 7%) [S. Ellairaja et al., ACS Sensors. 3 (2018) 2463-2466].

In literature there are reports on other systems for the fluorimetric determination of creatinine concentration. For example, there is known a method based on increasing the emission of rhodamine complex with gold(III) ions in the presence of creatinine [S. Ellairaja et al., ChemistrySelect 2 (2017) 1025-1031], and a method employing an interaction of creatinine with quantum dots coated with thioglycolic acid [N. Tajarrod et al., Anal. Methods 8 (2016) 5911-5920].

An alternative type of creatinine determinations in body fluids are enzymatic methods. This group of analytical methods provides much greater selectivity in effect of the substrate specificity of the enzyme used. In such methods, the analyte is converted by one or more enzymatic reactions into a product that can be easily determined by spectroscopic or electrochemical techniques.

The only method of enzymatic creatinine determination, which apart from the Jaffé method, is routinely used in clinical laboratories, is the colorimetric method for the determination of hydrogen peroxide resulting from a cascade of enzymatic reactions:

creatinine + H₂O -^{EC 3.5.2.10} -→ creatine

creatine + H₂O -^{EC 3.5.3.3}-→ urea + sarcosine

sarcosine + O₂ + H₂O -^{EC 1.5.3.1}-→ glycine + H₂O₂

The hydrogen peroxide is converted into the absorbing compound by another enzymatic reaction, catalysed by peroxidase (EC 1.11.1.7) in the presence of an acceptor. The absorbance of the reaction product is measured at 510 nm. The linear range of the method is up to 2210 µmol·L⁻¹ creatinine and the incubation time is 30 minutes. The method is characterised by high selectivity, however, in order to eliminate interference caused by ascorbic acid, the addition of ascorbate oxidase is necessary, while the addition of potassium ferrocyanide reduces the reduction of the signal caused by the presence of bilirubin [P. Fossati et al., Clin. Chem. 29 (1983) 1494-1496]

Currently, a modified version of the above method is used in routine clinical analysis. 3,5-Dichloro-2-hydroxybenzenesulfonic acid is replaced by a more effective hydrogen peroxide scavenger: 2,4,6-triiodo-3-hydroxybenzoic acid. The product of the enzymatic reaction is determined colorimetrically at a wavelength of 552 nm. The method has a creatinine detection limit of 5 µmol·L⁻¹, while its linear range is 2700 µmol·L⁻¹. The precision and repeatability, expressed as relative standard deviation, are less than 3%. The selectivity of the colorimetric enzyme method is much betterthan that of the Jaffé method. No influence of bilirubin (up to 324 µmol·L⁻¹), hemoglobin (up to 497 µmol·L⁻¹), ascorbic acid (up to 1700 µmol·L⁻¹), creatine (up to 3.05 mmol·L⁻¹) and most drugs for the results obtained has been noticed.

Isotope Dilution Mass Spectrometry (IDMS) is considered a completely selective (i.e. specific) method for determination of creatinine. The measurement procedure consists in enriching the sample with ¹³C-labeled creatinine. The creatine is then separated from the sample using a weal< acid ion exchange resin column and the creatinine is converted to N-(4,6-dimethyl-2-pyrimidinyl)-N-methylglycine ethyl ester. The next step is the analysis by mass spectrometry of the intensity ratio of peaks related to the creatinine derivative being not enriched and the isotopically enriched creatinine derivative [M. J. Welch et al., Anal. Chem. 58 (1986) 1681-1685]. The method is characterised by a very high precision and selectivity and is currently considered the reference method for determining creatinine. Unfortunately, the measurement of creatinine concentration by this method is very laborious and takes more than 24 hours (including sample preparation), and that is why it is not used routinely.

Another available method for determining creatinine is capillary electrophoresis, employing differences in the rate of compounds migration in an electric field.

It is also known to use high-performance liquid chromatography to determine the concentration of creatinine in biological samples. Creatinine is separated from the other sample components on a Nucleosil 120-3 C18 packed column. A mixture of water and acetonitrile (5% v/v acetonitrile) with the addition of sodium salt of octane sulfonic acid is used as an eluent. The latter compound acts as a reagent for the formation of ion pairs, being recommended due to the fact that that creatinine is a cation under the conditions of the measurement. Spectrophotometric detection is made at a wavelength of 236 or 215 nm. In such a system, the creatinine retention time is 7.26 minutes. The limit of creatinine determination under optimised conditions is 82 pmol·L⁻¹. This method has been proven effective in urine and blood serum samples. None of the other compounds present in such samples is eluted together with creatinine [D. Tsikas et al., Clin. Chem. 50 (2004) 201-203).

The publication No. EP 0066552 A1 discloses a method for determining creatinine content or concentration in biological samples, wherein creatinine is enzymatically transformed to glycine, acetaldehyde and hydrogen peroxide and proportioning one of the reaction end products. It is possible to proportionate hydrogen peroxide enzymatically in the presence of compounds giving a chromogen compound effective to be colourimetrically analysed.

The US 2019/219589 A1 reveals methods for measuring analyte alone or in combination with total protein in biological samples using one or more colorimetric reagents alone or in combination with protein precipitation reagents. For determination of creatinine in a urine sample an aqueous solution of 3,5-dinitrobenzoylchloride in phosphate buffer (pH 12.4) or alternatively hydrogen peroxide and an oxidizable dye are used to obtain a fluorophore being further processed. A combination of both reagents, *i.e.,* 3,5-dinitrobenzoilchloride and hydrogen peroxide is not disclosed.

The reaction of p-dinitrobenzene with H₂O₂ and NaOH in 10%, 30%, and 50% aqueous dioxane carried out at 30°C, involving formation of a reasonably stable intermediate which absorbs strongly in the visible region, has been an object of kinetic studies [A. Raymond et al., Can. J. Chem. 65 (1987) 251-255]. Although in the reaction mixture fluorophore is formed it is not suitable as a multi-compound fluorimetric reagent due to the fact that formation of fluorophore takes place immediately after mixing the p-dinitrobenzene with the remaining components and is not suitable for storage.

A fluorescent kidney function test based on reactivity of creatinine with alkaline 3,5-dinitrobenzoate has been reported back in 1995 [K. G. Blass, Christina et al., Clinical Biochemistry, Elsevier Inc., US, CA, vol.28, no. 2, (1995-0101), pp 107-111, XP002070749]. A simple, sensitive and highly specific fluorimetric method for the creatinine determination has been reported. A fluorophore was produced in the reaction of creatinine with 3,5-dinitrobenzoate under alkaline reaction conditions in aqueous and mixed solvent environment. The fluorophore had excitation and emission maxima near 410 and 475 nm, respectively. The calibration curve was linear in the range of creatinine concentration of 5-50 µmol·L⁻¹, while the detection limit for creatinine was well below 1 µmοl·L⁻¹.

In view of the absence in the prior art of an efficient, reliable and selective method of creatinine determination, that would involve additionally low analysis costs, it is the aim of the present invention to provide a new analytical method based to the maximum extent on known reagents, to be carried out using an optical detection and equipment commonly used in analytical laboratories, especially in clinical analytical laboratories.

This object is achieved in accordance with the invention, comprising a method for determining creatinine in clinically significant samples and a reagent to be used in the method.

### Summary of the invention

A fluorimetric analytical method for the determination of creatinine in clinically significant biological samples, in which creatinine is reacted with 3,5-dinitrobenzoic acid in a strongly alkaline aqueous-organic medium to form a fluorophore product, is characterised in that a clinically significant biological sample is mixed directly with a multi-component fluorimetric reagent containing 3,5-dinitrobenzoate anions, an organic solvent, water, a base maintaining alkalinity of the reagent above pH = 12, and an addition of hydrogen peroxide, whereas detection and/or quantification of the obtained product of creatinine reaction with 3,5-dinitrobenzoate anions is carried out using the fluorimetric method, by taking a measurement of radiation having a wavelength of 450-500 nm, preferably 480 nm, emitted under the influence of an excitation radiation beam of a wavelength of 380-420 nm, preferably 405 nm, at a constant incubation time after the start of the reaction and determining creatinine concentration on the basis of separately prepared calibration curve that links creatinine concentration in the biological sample with the fluorimetric response characteristic for the given wavelength and incubation time.

According to the invention, as a component containing 3,5-dinitrobenzoate anions, a solution of 3,5-dinitrobenzoic acid, a 3,5-dinitrobenzoate salt, preferably sodium 3,5-dinitrobenzoate or 3,5-dinirobenzoic acid ester, preferably methyl 3,5-dinitrobenzoate is used, the solution comprising an organic solvent, a mixture of organic solvents, water, or a mixture thereof. A solvent which is highly miscible with water, preferably a monohydric alcohol or a polyhydric alcohol is used as the organic solvent, wherein as the monohydric alcohol, methanol, ethanol, propanol or butanol is used, and 1,4-butanediol, 1,2-propanediol or ethylene glycol is used as the polyhydric alcohol, 1,4-butanediol being most preferred. A solution having a concentration greater than 0.05 mol·L⁻¹ is used as the base-containing component, which solution comprises water, an organic solvent or a mixture of organic solvents, or a mixture thereof, wherein as a base, a metal hydroxide, ammonium hydroxide or an organic base that does not significantly interfere with the fluorescence of the reaction product of creatinine with a 3,5-dinitrobenzoate anion, preferably an alkali metal hydroxide (LiOH, NaOH, KOH, RbOH, CsOH), ammonium hydroxide, tetramethylammonium hydroxide, tetraethylammonium hydroxide, tetrabutylammonium hydroxide, or mixtures thereof, most preferably LiOH, NaOH or KOH, is used. As a component containing hydrogen peroxide, an aqueous or aqueous-organic hydrogen peroxide solution is used.

According to the invention, the fluorimetric reagent is made of components including 3,5-dinitrobenzoic acid, 1,4-butanediol, water, NaOH and hydrogen peroxide, the components being mixed in any order and combination prior to adding the biological sample of clinical significance, wherein, stable component solutions are primarily prepared, preferably a solution containing 3,5-dinitrobenzoic acid, 1,4-butanediol, water and hydrogen peroxide, and a component solution containing water and NaOH, which component solutions are mixed immediately before adding the biological sample of clinical significance.

According to the invention, for samples containing creatinine in a concentration lower than or equal to 750 µmol·L⁻¹, the reagent is used in which:
- the concentration of 3,5-dinitrobenzoate ions ranges from 0.05 mmol·L⁻¹ up to saturation, preferably 12.5 mmol·L⁻¹,
- the base concentration is 0.05-2 mol·L⁻¹, preferably 1 mol·L⁻¹,
- the concentration of hydrogen peroxide is 1.45-145 mmol·L⁻¹, preferably 29 mmol·L⁻¹,
- the mutual ratio of solvents ranges from completely aqueous to aqueous-organic containing 0.3% water, and the optimal ratio of organic solvent to water in the water-organic mixture is from 0.8:1, while the samples containing creatinine in concentration greater than 750 µmol·L⁻¹ are diluted to a concentration lower than or equal to 750 µmol·L⁻¹

According to the invention, the volume ratio of the fluorimetric reagent to the clinically significant biological sample is from 1:1 to 299:1, preferably 14:1. The measurement of the fluorescence intensity is performed in the range of the observed linear dependence of the fluorescence intensity on the creatinine concentration, i.e. after the time of 20-3600 s, preferably 300 s. The creatinine determination range is within the range of 0.5-1000 µmol·L⁻¹ for the incubation time of 20-3600 s, preferably 3-750 µmol·L⁻¹ for the incubation time of 300 s.

The method according to the invention is selective for creatinine in the presence of sugars, proteins and bilirubin. A clinically significant biological sample is a blood serum at a 2-10-fold dilution, preferably at 5-fold dilution; a urine at a 10-400-fold dilution, preferably at 50-fold dilution; or a dialysate at a 1-5-fold dilution, preferably at 2-fold dilution.

A multi-component fluorimetric reagent, being alkaline and containing 3,5-dinitrobenzoate anions in an aqueous-organic environment, characterised in that it additionally contains hydrogen peroxide and 1,4-butanediol, is suitable for use in the analytical fluorimetric method for determination of creatinine in clinically significant biological samples, and after direct mixing with a clinically significant biological sample containing creatinine, selectively produces a fluorophore in the reaction between creatinine and a 3,5-dinitrobenzoate anion, which fluorophore under the influence of an excitation radiation beam of a wavelength of 380-420 nm, preferably 405 nm, emits radiation of a wavelength of 450-500 nm, preferably 480 nm, wherein the reagent contains 3,5-dinitrobenzoate ions at a concentration of from 0.05 mmol·L⁻¹ up to saturation, preferably 12.5 mmol·L⁻¹; the base at a concentration of 0.05-2 mol-L¹, preferably 1 mol·L⁻¹; hydrogen peroxide at a concentration of 1.45-145 mmol·L⁻¹, preferably 29 mmol·L⁻¹; and the mutual ratio of the solvents ranges from completely organic to fully aqueous, and the optimal ratio of organic solvent to water in the aqueous-organic mixture is from 0.8:1, which fluorimetric reagent is stable at room temperature for over 100 hours after its preparation and can be used for the selective determination of creatinine at concentrations ranging from 3-750 µmol·L⁻¹ in the presence of glucose, proteins and bilirubin, for an incubation time of 20-3600 s, preferably 300 s.

When using the solution according to the invention, it is possible to accurately and precisely determine the concentration of creatinine in biological samples. The results obtained with the method according to the invention are consistent with the results obtained in the Clinical Laboratory, which routinely uses the enzymatic method, which requires the use of as many as four enzymes and a number of other reagents. Enzyme preparations are relatively expensive and must be stored in refrigerators at low temperatures, and in solutions are often quite unstable. In addition, a decisive difficulty of polyenzymatic methods is to find the optimal reaction conditions, especially the pH of the solution and the temperature at which all enzymes work satisfactorily. Overcoming these drawbacks by using the simple, one-point method according to the invention is very attractive, that being its most important advantage over the prior art methods for creatinine determination.

It is also an advantage of the present invention that the measurement in the disclosed method is a single point one and the reagents are non-toxic, which allows for the development of mobile measurement systems. Using the method according to the invention, it is possible to create systems ready to use at any time and place, in accordance with the concept of the *point of care testing,* i.e. carrying out analytical tests directly at the place of patient care, for example at the bedside in the hospital, in ambulances or in the offices of primary contact (GP) doctors, by personnel not being trained in clinical analytics.

The possibility of abandoning the two-point measurement in the practice of analytical laboratories is a great help in routine determinations, and most importantly, it reduces the measurement uncertainty. In the case of the two-point measurement, the analytical signal is a difference of two values, both of which are subject to measurement uncertainty. In the simplest terms, the error of the difference between the two values will be the square root of the sum of the squared errors of the two measurements, therefore the obtained error value is greater than that of the single-point measurement.

When using the solution according to the invention, it is possible to accurately and precisely determine the concentration of creatinine in biological samples. The results obtained with the method according to the invention are consistent with the results obtained in the Clinical Laboratory, which routinely uses the enzymatic method, which requires the use of as many as four enzymes and a number of other reagents. Enzyme preparations are relatively expensive and must be stored in refrigerators at low temperatures, and in solutions are often quite unstable. In addition, a decisive difficulty of polyenzymatic methods is to find the optimal reaction conditions, especially the pH of the solution and the temperature at which all enzymes work satisfactorily. Overcoming these drawbacks by using the simple, one-point method according to the invention is very attractive, that being its most important advantage over the prior art methods for creatinine determination.

It is also an advantage of the present invention that the measurement in the disclosed method is a single point one and the reagents are non-toxic, which allows for the development of mobile measurement systems. Using the method according to the invention, it is possible to create systems ready to use at any time and place, in accordance with the concept of the *point of care testing,* i.e. carrying out analytical tests directly at the place of patient care, for example at the bedside in the hospital, in ambulances or in the offices of primary contact (GP) doctors, by personnel not being trained in clinical analytics.

The possibility of abandoning the two-point measurement in the practice of analytical laboratories is a great help in routine determinations, and most importantly, it reduces the measurement uncertainty. In the case of the two-point measurement, the analytical signal is a difference of two values, both of which are subject to measurement uncertainty. In the simplest terms, the error of the difference between the two values will be the square root of the sum of the squared errors of the two measurements, therefore the obtained error value is greater than that of the single-point measurement.

The invention will now be described in detail by way of example with reference to the accompanying drawing. The values in the graph correspond to the final concentrations in the reagent.

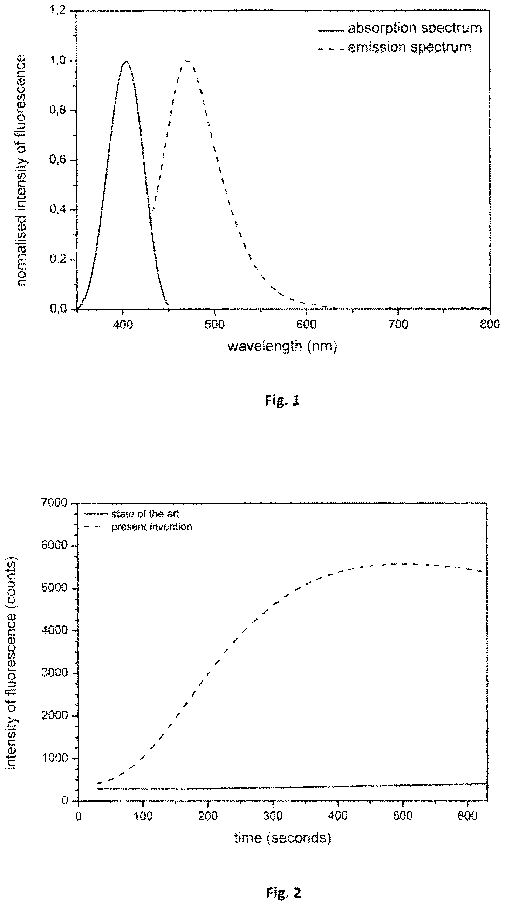

- Fig. 1: shows the excitation and emission spectra of the creatinine - 3,5-dinitrobenzoic acid (DNBA) reaction product dissolved in 1,4-butanediol (BTD). Creatinine (250 µmol·L⁻¹), DNBA (12.5 mmol·L⁻¹), NaOH (1 mol·L⁻¹), H₂O₂ (29 mmol·L⁻¹), V_{BTD}/V_{H₂O} = 0.8:1.
- Fig. 2: shows a comparison of the dependence of the fluorescence intensity of the creatinine-DNBA reaction product on time for the process carried out without the addition of H₂O₂ according to the K.G. Blass protocol [Clin. Biochem. 28 (1995) 107-111] and for the process with the addition of 29 mmol·L⁻¹ H₂O₂ according to the present invention. Excitation wavelength λₑₓ = 405 nm, emission wavelength λₑₘ = 480 nm. H₂O₂ concentration in water: C°_{H₂O₂} = 580 mmol·L⁻¹. Component concentrations in the reagent: DNBA (12.5 mmol·L⁻¹), NaOH (1 mol·L⁻¹), 1,4-butanediol to water volume ratio: V_{BTD}/V_{H₂O} = 0.8:1. Creatinine concentration in the standard (250 µmol·L⁻¹). The ratio of the volume of the fluorimetric reagent to the sample volume: V_{REAGENT}/V_{SAMPLE} = 14:1.

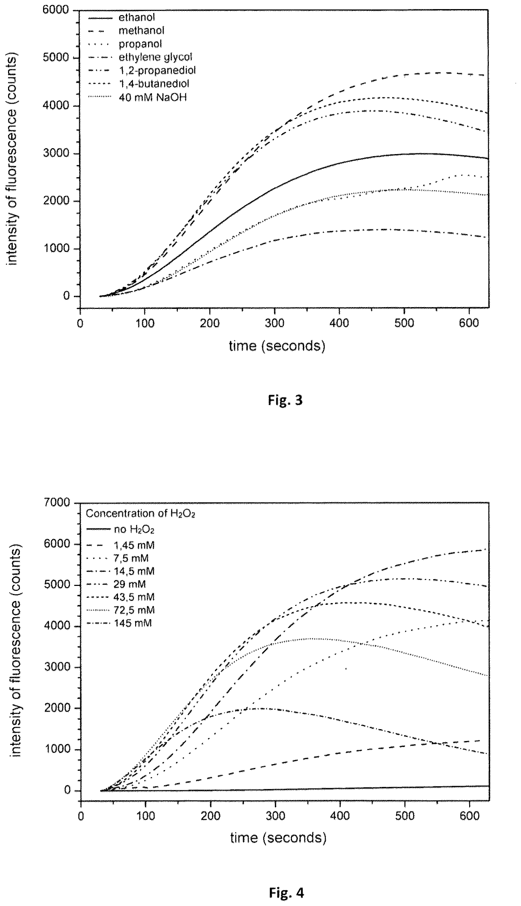

- Fig. 3: shows the dependence of the fluorescence intensity of the creatinine-DNBA reaction product on time for the different solvents used to dissolve DNBA. λₑₓ = 405 nm, λₑₘ = 480 nm. Reagent: DNBA (12.5 mmol·L⁻¹), NaOH (1 mol·L⁻¹), H₂O₂ (29 mmol·L⁻¹), V_{SOLVENT}/V_{H2O} = 0.8:1. Creatinine standard solution (250 µmol·L⁻¹), V_{REAGENT}/V_{SAMPLE} = 14:1. n = 3.
- Fig. 4: shows the dependence of the fluorescence intensity of the creatinine-DNBA reaction product on time for various concentrations of H₂O₂ in the reagent in the range of 0-145 mmol·L⁻¹. λₑₓ = 405 nm, λₑₘ = 480 nm. Reagent: DNBA (13.5 mmol·L⁻¹), NaOH (1 mol·L⁻¹), V_{SOLVENT}/ V_{H₂O} = 0.8:1. Creatinine standard solution (250 µmol·L⁻¹). V_{REAGENT}/V_{SAMPLE} = 14:1.

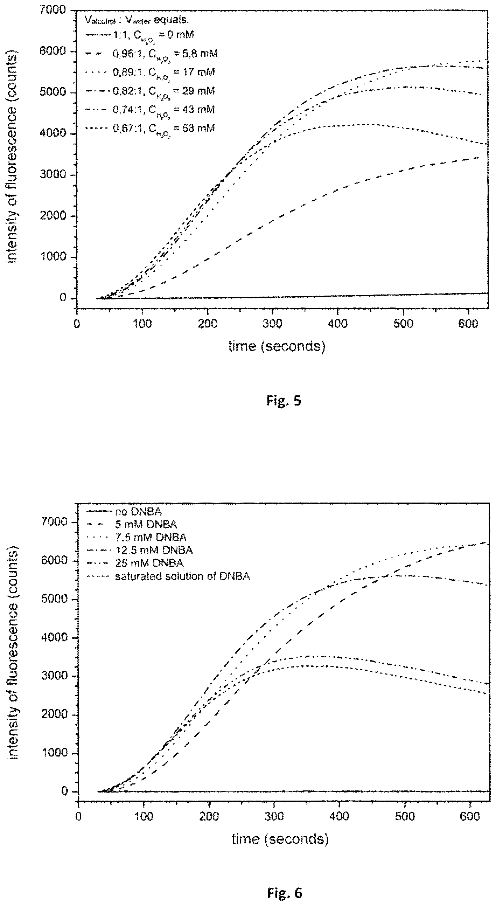

- Fig. 5: shows the dependence of the fluorescence intensity of the creatinine-DNBA reaction product for different volume ratios of BTD to aqueous solution of H₂O₂ in a fluorimetric reagent ranging from 4:1 to 49:1, and also in the absence of H₂O₂. The range of changes in the concentration of H₂O₂ in the fluorimetric reagent is 0-58 mmol·L⁻¹. The range of V_{BTD}/V_{H₂O} changes in the fluorimetric reagent ranges from 1:1 to 0.67:1. λₑₓ = 405 nm, λₑₘ = 480 nm. C°_{H₂O₂} = 580 mmol·L⁻¹. Reagent: DNBA (13.5 mmol·L⁻¹), NaOH (1 mol·L⁻¹). Creatinine standard solution (250 µmol·L⁻¹). V_{REAGENT}/V_{SAMPLE} = 14:1.
- Fig. 6: shows the relationship of the fluorescence intensity of the creatinine-DNBA reaction product over time for different concentrations of DNBA in the fluorimetric reagent from zero to saturation. λₑₓ = 405 nm, λₑₘ = 480 nm. Reagent: NaOH (1 mol·L⁻¹), H₂O₂ (29 mmol·L⁻¹), V_{BTD}/V_{H₂O} = 0.8:1. Creatinine standard solution (250 µmol·L⁻¹). V_{REAGENT}/V_{SAMPLE} = 14:1

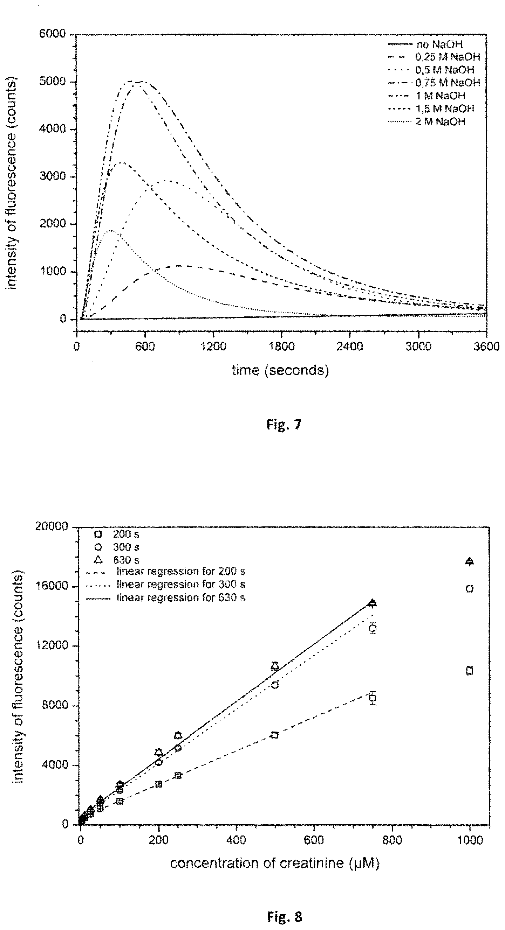

- Fig. 7: shows the dependence of the fluorescence intensity of the creatinine-DNBA reaction product on time for various NaOH concentrations in the range of 0-2 mol·L⁻¹. λₑₓ = 405 nm, λₑₘ = 480 nm. Reagent: DNBA (12.5 mmol·L⁻¹), H₂O₂ (29 mmol·L⁻¹). V_{BTD}/V_{H₂O} = 0.8:1. Creatinine standard solution (250 µmol·L⁻¹). V_{REAGENT}/V_{SAMPLE} = 14:1.
- Fig. 8: shows the calibration characteristics of the method for determining creatinine in an aqueous-organic environment according to the invention for a creatinine concentration in the range of 0-1000 µmol·L⁻¹, for reaction times: 200 s, 300 s and 630 s. V_{REAGENT}/V_{SAMPLE} = 14:1. The number of repetitions of the measurement: n = 3.

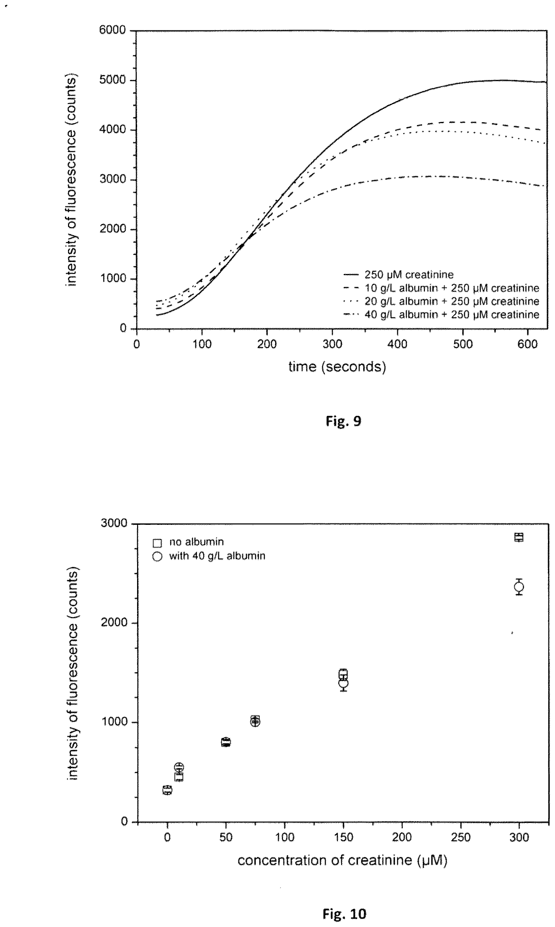

- Fig. 9: shows the dependence of the fluorescence intensity of the creatinine-DNBA reaction product on time for the albumin concentration in the range of 0-40 g·L⁻¹. λₑₓ = 405 nm, λₑₘ = 480 nm. Reagent: DNBA (12.5 mmol·L⁻¹), H₂O₂ (29 mmol·L⁻¹). V_{BTD}/V_{H₂O} = 0.8:1. Creatinine standard solution (250 µmol·L⁻¹). V_{REAGENT}/V_{SAMPLE} = 14:1. n = 3.
- Fig. 10: shows the calibration characteristics of the method of creatinine determination according to the invention for the concentration of creatinine in the range of 0-300 µmol·L⁻¹, for pure solutions and with the addition of albumin at a concentration of 40 g·L⁻¹, for the measurement time: 200 s. λₑₓ = 405 nm, λₑₘ = 480 nm. Reagent: DNBA (12.5 mmol·L⁻¹), H₂O₂ (29 mmol·L⁻¹). V_{BTD}/V_{H₂O} = 0.8:1. Creatinine standard solution (250 µmol·L⁻¹). V_{REAGENT}/V_{SAMPLE} = 14:1. n = 3.

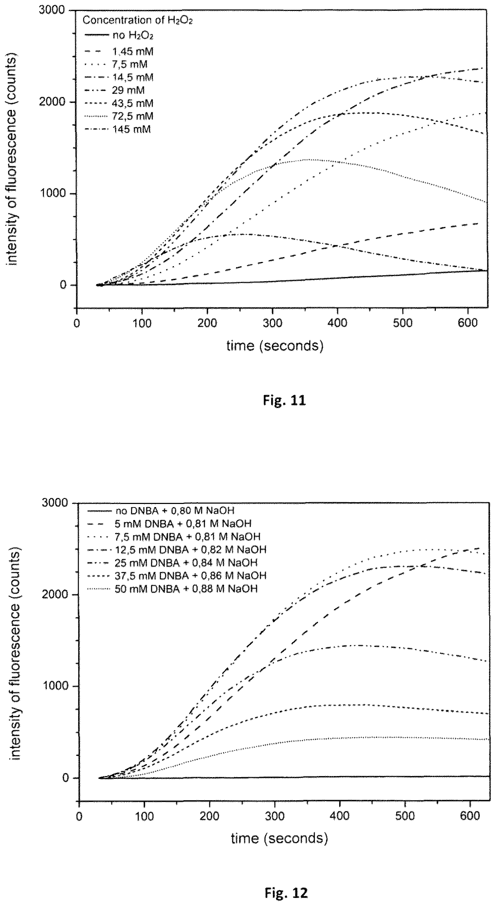

- Fig. 11: shows the relationship of the fluorescence intensity of the creatinine-DNBA reaction product over time in the water environment for various concentrations of H₂O₂ in the fluorimetric reagent in the range of 0-145 mmol·L⁻¹. λₑₓ = 405 nm, λₑₘ = 480 nm. Reagent: DNBA (13.5 mmol·L⁻¹), NaOH (0.8 mol·L⁻¹). Creatinine standard solution (250 µmol·L⁻¹). V_{REAGENT}/V_{SAMPLE} = 14:1.
- Fig. 12: shows the relationship of the fluorescence intensity of the creatinine-DNBA reaction product over time in the water environment for various concentrations of DNBA in the fluorimetric reagent in the range of 0-50 mmol·L⁻¹. λₑₓ = 405 nm, λₑₘ = 480 nm. Reagent: NaOH (0.8 mol·L⁻¹), H₂O₂ (29 mmol·L⁻¹). Creatinine standard solution (250 µmol·L⁻¹). V_{REAGENT}/V_{SAMPLE} = 14:1.

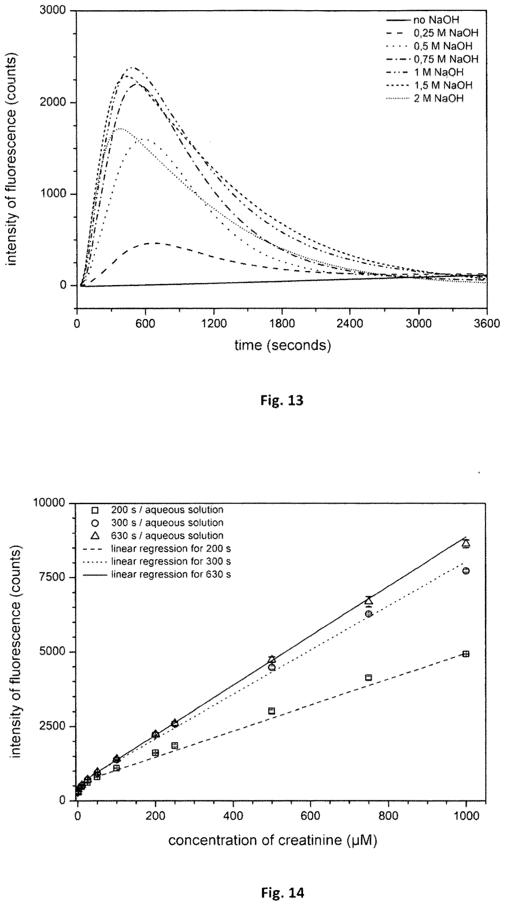

- Fig. 13: shows the dependence of the fluorescence intensity of the creatinine-DNBA reaction product on time for various concentrations of NaOH in the range of 0-2 mol·L⁻¹. λₑₓ = 405 nm, λₑₘ = 480 nm. Reagent: DNBA (7.5 mmol·L⁻¹), H₂O₂ (29 mmol·L⁻¹). Creatinine standard solution (250 µmol·L⁻¹). V_{REAGENT}/V_{SAMPLE} = 14:1.
- Fig. 14: shows the calibration characteristics of the method for the determination of creatinine in an aqueous medium according to the invention for a creatinine concentration in the range of 0-1000 µmol·L⁻¹, for reaction times: 200 s, 300 s and 630 s. V_{REAGENT}/V_{SAMPLE} = 14:1. n = 3.

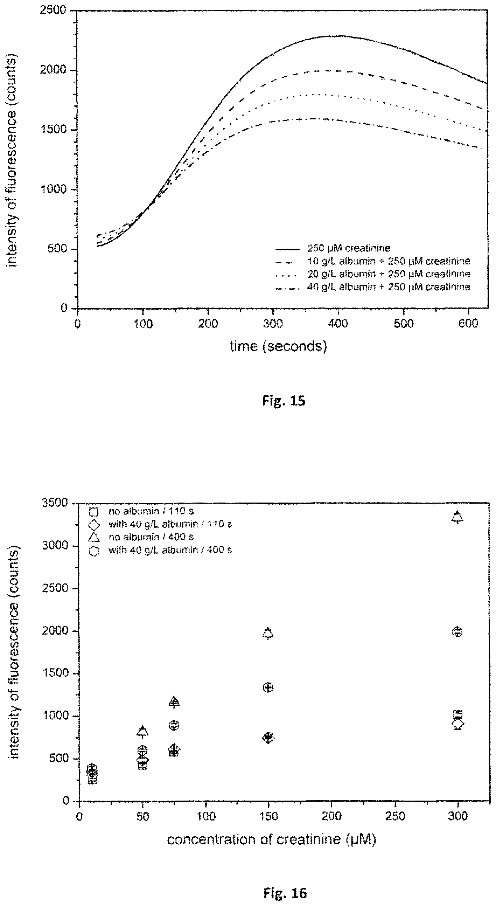

- Fig. 15: shows the relationship of the fluorescence intensity of the creatinine-DNBA reaction product over time for albumin concentration in the range of 0-40 g·L⁻¹. λₑₓ = 405 nm, λₑₘ = 480 nm. Reagent: DNBA (7.5 mmol·L⁻¹), H₂O₂ (29 mmol·L⁻¹). Creatinine standard solution (250 µmol·L⁻¹). V_{REAGENT}/V_{SAMPLE} = 14:1. n = 3.
- Fig. 16: shows the calibration characteristics of the method for determining creatinine according to the invention in an aqueous medium for a creatinine concentration in the range of 0-300 µmol·L⁻¹, for pure solutions and with the addition of albumin at a concentration of 40 g·L⁻¹, for the measurement time: 110 s and 400 s. λₑₓ = 405 nm, λₑₘ = 480 nm. Reagent: DNBA (7.5 mmol·L⁻¹), H₂O₂ (29 mmol·L⁻¹). Creatinine standard solution (250 µmol·L⁻¹). V_{REAGENT}/V_{SAMPLE} = 14:1. n = 3.

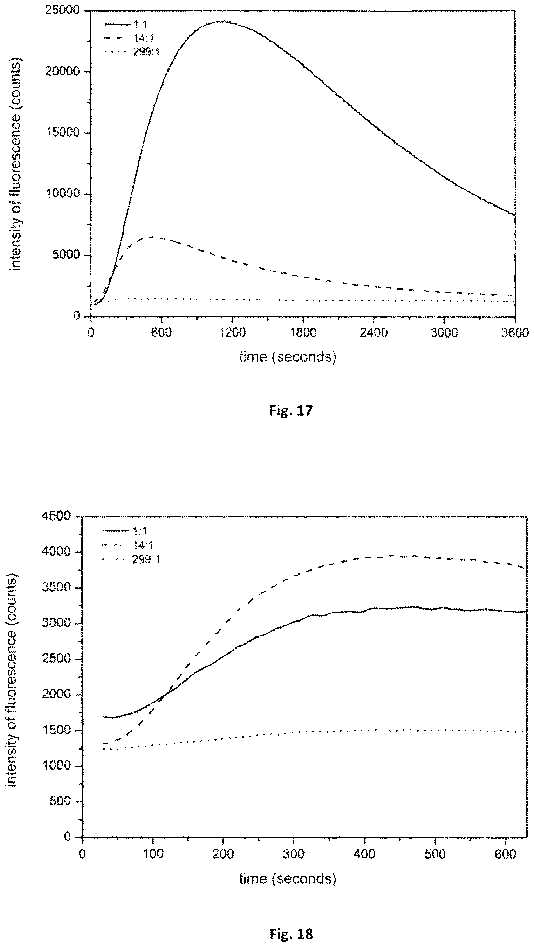

- Fig. 17: shows a comparison of the fluorescence intensity of the creatinine-DNBA reaction product over time for various reagent to sample volume ratios: 1:1, 14:1 and 299:1. λₑₓ = 405 nm, λₑₘ = 480 nm. Reagent: DNBA (12.5 mmol·L⁻¹), H₂O₂ (29 mmol·L⁻¹). V_{BTD}/V_{H₂O} = 0.8:1. Creatinine standard solution (250 µmol·L⁻¹). n = 3.
- Fig. 18: shows a comparison of the fluorescence intensity of the creatinine-DNBA reaction product over time in the presence of albumin at a concentration of 40 g·L⁻¹ for various reagent to sample volume ratios: 1:1, 14:1 and 299:1. λₑₓ = 405 nm, λₑₘ = 480 nm. Reagent: DNBA (12.5 mmol·L⁻¹), H₂O₂ (29 mmol·L⁻¹). V_{BTD}/V_{H₂O} = 0.8:1. Creatinine standard solution (250 µmol·L⁻¹). V_{REAGENT}/V_{SAMPLE} = 14:1. n = 3.

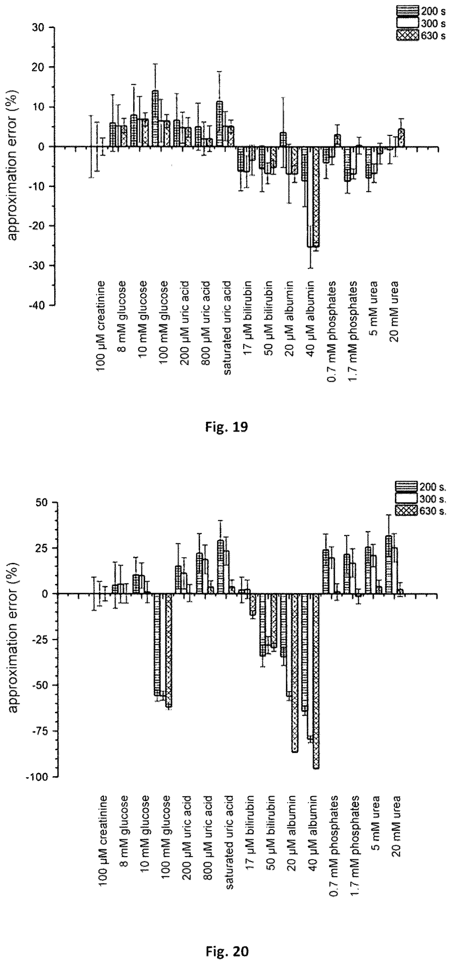

- Fig. 19: shows the interferences of the fluorimetric method of creatinine determination according to the invention using V_{REAGENT}/V_{SAMPLE} = 14:1. λₑₓ = 405 nm, λₑₘ = 480 nm. Reagent: DNBA (12.5 mmol·L⁻¹), H₂O₂ (29 mmol·L⁻¹). V_{BTD}/V_{H₂O} = 0.8:1. Creatinine standard solution (250 µmol·L⁻¹). n = 3.
- Fig. 20: shows the interferences of the fluorimetric method of creatinine determination according to the invention when V_{REAGENT}/V_{SAMPLE} = 1:1. λₑₓ = 405 nm, λₑₘ = 480 nm. Reagent: DNBA (25 mmol·L⁻¹), H₂O₂ (44 mmol·L⁻¹). V_{BTD}/V_{H₂O} = 0.8:1. Creatinine standard solution (100 µmol·L⁻¹). n = 3.

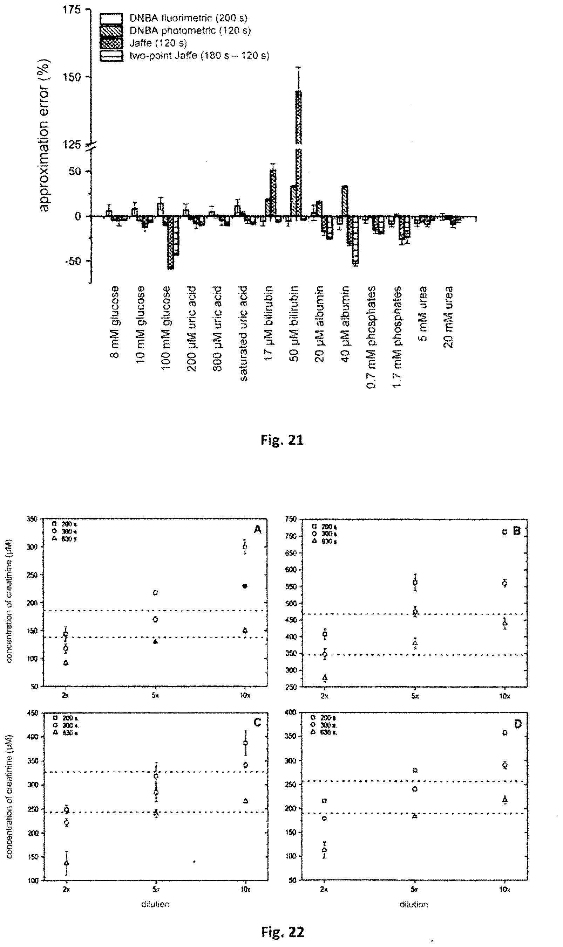

- Fig. 21: shows a comparison of the selectivity of the creatinine determination method according to the invention with the photometric method using DNBA and with the Jaffé methods.
- Fig. 22: shows the results of the determination of creatinine in synthetic serum employing the method according to the invention for different sample dilutions (2x, 5x, 10x) and incubation time (200-630 s). A - HN serum, B - HP serum, C - 1:1 HN/HP mixture, D - 3:1 HN/HP mixture. The declared range of concentrations is marked with dashed lines.

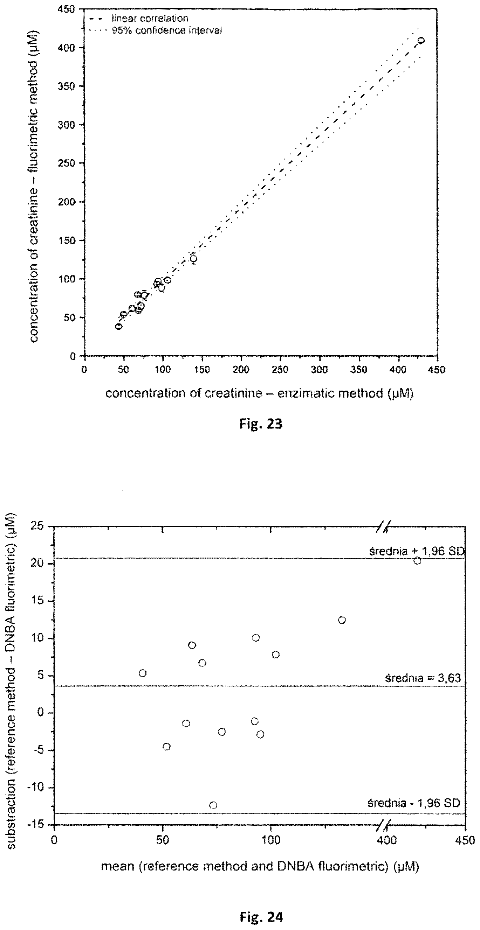

- Fig. 23: shows the degree of correlation between the results obtained employing the creatinine determination method according to the invention and the results obtained in the Central Clinical Laboratory using the enzymatic method. 95% confidence interval. n = 3.
- Fig. 24: is a Bland-Altman plot showing the agreement between the results obtained when employing the method of the invention and the results obtained when using the enzymatic method.

### Detail description of the invention

Despite the long history of determinations of creatinine in clinical biological samples, it is still a significant problem to find a reliable, cheap and convenient analytical method for this purpose, which would be characterised by greater reliability than the routine Jaffé method, as well as lower price and lower complexity than the routinely used enzymatic methods. This is evidenced, for example, by numerous reviews and research papers published in recent years on the subject, that may be related to the ever-increasing number of people suffering from chronic kidney disease (25% increase in the incidence in 2005-2015) [Lancet 388 (2016) 1545-1602].

The research on the present invention was inspired by the desire to provide a method of high reliability (selective against other components of biological samples), which would be easy, fast and cheap to be prepare. As a result, the new method would be competitive to the Jaffé method, which is highly susceptible to interferences, and to the enzymatic methods, the disadvantageous due to the need of using expensive preparations with limited durability and to the need of using long incubation times.

Research has focused on the development of a fluorimetric method for determining creatinine in serum samples, although the analysis of creatinine concentration in urine and dialysate is also very important, mainly because creatinine is used as an indicator of urine dilution and is proportional to the patient's muscle mass. In addition, urine and dialysate have a much poorer matrix than blood serum, they should not contain proteins and bilirubin, the level of creatinine in the urine is 2 orders of magnitude higher than in the blood serum and the level of creatinine in the dialysate is similar to the level of creatinine in the blood serum.

However, the differences in the characteristics of the above-mentioned biological samples do not affect the applicability of the creatinine determination method according to the invention, which may be successfully applied to all those types of samples, just taking into account the necessary dilutions to ensure optimal creatinine content in the analysed samples.

Literature reports indicated that it is possible to determine creatinine fluorimetrically on the basis of the creatinine reaction with 3,5-dinitrobenzoic acid [K. G. Blass, Clin. Biochem. 28 (1995) 107-111], however, so far no one has reproduced the results. In the course of the research related to the present invention, it was proven that the previously described method does not work even under the proposed conditions and that it is not possible to obtain a fluorimetric signal even when larger amounts of the analyte are used (Fig. 2).

### The role of hydrogen peroxide

In the course of the current research, however, a significant analytical potential of the fluorimetric method of creatinine determination based on its reaction with ions 3,5-dinitrobenzoate ions has been confirmed (the ions being introduced into the reaction system as a reagent comprising 3,5-dinitrobenzoic acid, a salt or an ester thereof).

The observation that allowed for further experiments was the finding that the presence of hydrogen peroxide in the reaction mixture leads to the formation of a fluorescent product in the creatinine reaction with 3,5-dinitrobenzoic acid dissolved in 1,4-butanediol in a strongly alkaline medium. The emission spectrum of the fluorophore after excitation by radiation with a wavelength of 405 nm (maximum of the absorption spectrum) is shown in Fig. 1. The maximum emission of the fluorophore occurs at a wavelength of 480 nm.

The key to the effectiveness of the creatinine determination method was the addition of hydrogen peroxide to the reaction environment, conditioning and enabling the occurrence of the fluorescence phenomenon in the presence of the product of creatinine reaction with 3,5-dinitrobenzoate ions. The use of H₂O₂ in the fluorescence determination of creatinine has not been previously known in the literature.

Although the mechanism of hydrogen peroxide action in the creatinine reaction with 3,5-dinitrobenzoate ions in an alkaline environment is still unknown, a reproducible and reliable effect is achieved. Currently, it has been excluded that hydrogen peroxide oxidises the solvent used to prepare the acid solution, because while it is possible to oxidise alcohols by peroxide, the reaction requires the presence of a catalyst in the form of sodium tungstate and tetraalkyl-ammonium hydrogen sulfate, a significantly increased concentration of hydrogen peroxide and the elevated temperature [N. Ryoji et al., Chem. Commun. 16 (2003) 1977-1986]. Moreover, the fluorescent reaction product is formed not only when hydrogen peroxide is previously added to the component solution containing 3,5-dinitrobenzoic acid and an organic solvent (*e.g*. 1,4-butanediol), but also when it is mixed with the component solution containing the base (*e.g*. LiOH, NaOH, KOH), and also when it is added separately just before the reaction. It was also confirmed that the replacement of the H₂O₂ addition with the enrichment of the reaction reagent with the solvent oxidation product (*e.g*. succinic acid, which is the 1,4-butanediol oxidation product) does not allow for fluorescence in the tested system.

Without wishing to limit the scope of possible explanations, it is necessary to mention the observation made, which may contribute to explaining the mechanism of the taking place reaction. For all tested NaOH concentrations, after approx. 20-30 seconds of incubation, the phase of intense signal increase begins, followed by a decrease in the fluorescence intensity to a value close to the signal corresponding to the blank sample, and after approx. 3600 s the fluorescence disappears (Fig. 7). This suggests that the fluorescent compound may be an intermediate product of the creatinine reaction with 3,5-dinitrobenzoate anions, which intermediate product is transformed after some time into a non-fluorescent final product.

### Assumptions on usability of the method

In the first phase, the conditions of the reaction between creatinine and 3,5-dinitrobenzoic ions, leading to the formation of the previously described product (complex/adduct) absorbing and/or emitting electromagnetic radiation, were investigated and optimised. Then, the calibration characteristics of the developed method and its analytical parameters, such as the limits of detection and quantification, linear range and precision, were determined. The selectivity of the method according to the invention was also tested and compared with the selectivity of the routinely used Jaffé method. The working examples demonstrate the use of the developed protocols for the determination of creatinine in serum, urine and dialysate samples.

In the course of the work, the main problem turned out to be a significant reduction of the analytical signal caused by the presence of interferents, especially proteins, in biological samples. In such cases, the most common remedy is the addition of denaturing agents to denature proteins, or the addition of a constant amount of protein to the calibrators. Unfortunately, in the case of the method according to the invention, no effective denaturing agent was found, which does not exclude the possibility of developing such an agent in future. In turn, the possible addition of protein to the calibration solutions would significantly deteriorate the sensitivity of the reaction, because the presence of even small amounts of albumin (20 g·L⁻¹) causes a decrease in the fluorescence of the reaction according to the invention by as much as 90%. This effect can be minimised by significant (*e.g*. 50-fold) dilution of biological samples, which is, however, inconvenient and practically excludes the possibility of carrying out measurements in accordance with the concept of *point of care testing,* i.e. conducting analytical tests directly at the place of patient care - at the bedside in hospitals, in ambulances or in GP practices by staff not being trained in clinical analytics.

### Optimisation of the fluorimetric method of creatinine determination in biological samples

The optimisation of the method according to the invention consisted in determining the optimal composition of a fluorimetric reagent, containing all the necessary components for the creatinine reaction with the 3,5-dinitrobenzoate anion.

During the optimisation experiments, the following were determined:
- optimal concentration range of 3,5-dinitrobenzoate ions,
- optimal concentration range of the base maintaining the pH of the reaction mixture above 12,
- optimal range of hydrogen peroxide concentration,
- optimal volume ratio of organic solvent to water,
- optimal volume ratio of the fluorimetric reagent to a biological sample.

During optimisation experiments, a fluorimetric reagent was produced using mainly 3,5-dinitrobenzoic acid, 1,4-butanediol, water, hydrogen peroxide and sodium hydroxide, due to their easy availability and low cost. Typically, a component solution containing 3,5-dinitrobenzoic acid, 1,4-butanediol, water and hydrogen peroxide at appropriate concentrations was formed and mixed with the component solution containing NaOH just prior to the experiment.

However, the established procedure does not exclude replacing of the above components with their chemical equivalents, which will play an identical function in the fluorescence process of the creatinine-DNBA reaction product. The task of the 3,5-dinitrobenzoic acid compound is to form a complex/adduct in the course of its reaction with creatinine. The purpose of the organic solvent is to facilitate the dissolution of the 3,5-dinitrobenzoic acid compound since, for example, 3,5-dinitrobenzoic acid is poorly soluble in water. However, this does not exclude carrying out measurements in a pure-water solution, as long as it ensured to be alkaline, which enables the formation of 3,5-dinitrobenzoate anions. The role of the base is to provide an alkaline environment that stabilises the 3,5-dinitrobenzoate anions and enables the fluorescence process to take place, and in the case of measurements in an aqueous medium, also to facilitate the dissolution of the 3,5-dinitrobenzoate compound. The role of hydrogen peroxide is to enable the fluorescence process to take place.

According to the invention, it is possible to use 3,5-dinitrobenzoic acid, 3,5-dinitrobenzoate salt (e.g. sodium salt) or 3,5-dinirobenzoate ester (e.g. methyl 3,5-dinitrobenzoate) as the source of 3,5-dinitrobenzoate anions. Any organic solvent that dissolves the 3,5-dinitrobenzoate compounds and is highly miscible with water may be used. For example, monohydric alcohol (methanol, ethanol, propanol or butanol), polyhydric alcohol (1,4-butanediol, 1,2-propanediol or ethylene glycol) or mixtures thereof can be used. Any aqueous or aqueous-organic solution of a base in concentration above 0.05 mol·L⁻¹ can be used, as long as the base (organic or inorganic) does not significantly interfere with the fluorescence of the product of creatinine reaction with 3,5-dinitrobenzoate, such as for example alkali metal hydroxide (LiOH, NaOH, KOH, RbOH, CsOH), ammonium hydroxide, organic bases (tetramethylammonium hydroxide, tetraethylammonium hydroxide, tetrabutylammonium hydroxide) or mixtures thereof may be used. It is possible to use an aqueous or a water-organic hydrogen peroxide solution.

According to the invention, the fluorimetric reagent is prepared from suitable components which are mixed in any order and combination prior to addition of a clinically significant biological sample. The fluorescent reagent is stable at room temperature for more than 100 hours.

### Optimisation of the composition of the fluorimetric reagent containing organic solvent

Unless otherwise stated, in all fluorimetric measurements the operating voltage of the photomultiplier was 700 V, while the width of the slots for both the excitation beam and the emitted beam was 2.5 nm. A beam excitation with a wavelength of λₑₓ = 405 nm (maximum of the absorption spectrum of the creatinine-DNBA reaction product, Fig. 1) was used, and the fluorescence intensity was recorded for the wavelength λₑₘ = 480 nm (the maximum emission spectrum of the creatinine-DNBA reaction product, Fig. 1). In kinetic measurements, the signal was recorded every second. All optimisation runs are standardised. Scinco FluoroMate FS-2 spectrofluorimeter was used.

In order to select the optimal solvent for 3,5-dinitrobenzoate compounds, which are the main component of the fluorescent reagent, a series of measurements was performed. The criteria that must be met by an optimal solvent are: first of all miscibility with water, low volatility and low toxicity. Initially, monohydroxy alcohols (methanol, ethanol, propanol), polyhydroxy alcohols (ethylene glycol, 1,2-propanediol, 1,4-butanediol) and aqueous all<aline solutions were selected. A series of fluorimetric reagent solutions were prepared using pre-selected solvents in a ratio of 0.8:1 with water, containing DNBA (12.5 mmol·L⁻¹), NaOH (1 mol·L⁻¹), H₂O₂ (29 mmol·L⁻¹). After mixing the reagent with the standard solution of creatinine (250 µmol·L⁻¹) in a volume ratio of 14:1, the variability of the fluorescence intensity (λₑₘ = 480 nm) was recorded after excitation by irradiation of the wavelength λₑₓ = 405 nm. Measurable fluorescence was recorded in each of the tested alcohols. Among the solvents used, 1,4-butanediol allows for the fastest reaction between DNBA - and creatinine (maximum intensity approx. 4200 counts, after an incubation time of approx. 450 s). Methanol also seems to be a very promising solvent (4800 counts/520 s), but due to its volatility, there were large differences between the obtained results, and its toxicity could exclude it from applicability. Of all the solvents tested 1,2-propanediol could be an alternative for 1,4-butanediol (4000 counts/450 s) because it is non-toxic, readily available and inexpensive. The results are shown in Fig. 3.

In order to select the optimal concentration of H₂O₂ in the fluorimetric reagent, a series of reagent solutions containing DNBA (13.5 mmol·L⁻¹), NaOH (1 mol·L⁻¹) 1.4-butanediol and water in a volume ratio of 0.8:1, and H₂O₂ in various concentrations ranging from 0-145 mmol·L⁻¹ were prepared. After mixing the reagent with the creatinine standard solution (250 µmol·L⁻¹) in a 14:1 volume ratio, the dependence of the fluorescence intensity on time was investigated (λₑₓ = 405 nm, λₑₘ = 480 nm). The optimal kinetics (5000 counts/480 s) was observed for an H₂O₂ concentration of 29 mmol·L⁻¹. A slightly higher intensity of fluorescence was observed for the concentration of H₂O₂ 14.5 mmol·L⁻¹, but a longer incubation (6000 counts/700 s) was required, which would be burdensome with the routine use of the method. The results are shown in Fig. 4.

In order to select the optimal volume ratio of 1,4-butanediol to the component of the H₂O₂ solution in water (initial concentration C°_{H₂O₂} = 580 mmol·L⁻¹), a series of reagent solutions containing DNBA (13.5 mmol·L⁻¹), NaOH ( 1 mol·L⁻¹) were prepared. The range of changes in the concentration of H₂O₂ in the fluorimetric reagent was: 0-58 mmol·L⁻¹. The range of changes in the volume ratio 1,4-butanediol to water ranged from 1:1 to 0.67:1. After mixing the reagent with the standard creatinine solution (250 µmol·L⁻¹) in the volume ratio of 14:1, the dependence of fluorescence intensity on time was investigated (λₑₓ = 405 nm, λₑₘ = 480 nm). The most favorable volume ratio of BTD to H₂O₂ aqueous solution was found to be the ratio 9:1 (5800 counts/480 s). The results are shown in Fig. 5.

In order to select the optimal concentration of DNBA in the fluorimetric reagent, a series of reagent solutions containing NaOH (1 mol·L⁻¹), H₂O₂ (29 mmol·L⁻¹), 1,4-butanediol and water in the volume ratio of 0.8:1, and DNBA at various concentrations ranging from zero to saturation were prepared. After mixing the reagent with the creatinine standard solution (250 µmol·L⁻¹) in a volume ratio of 14:1, the dependence of the fluorescence intensity on time was investigated (λₑₓ = 405 nm, λₑₘ = 480 nm). The optimal kinetics (4500 counts/450 s) was observed for DNBA concentration of 12.5 mmol·L⁻¹. The results are shown in Fig. 6.

In order to select the optimal concentration of NaOH in the fluorimetric reagent ensuring maximum sensitivity of the method, a series of reagent solutions containing DNBA (12.5 mmol·L⁻¹), H₂O₂ (29 mmol·L⁻¹), 1,4-butanediol and water in the volume ratio of 0.8:1, and NaOH at various concentrations ranging from 0-2 mol·L⁻¹ were prepared. After mixing the reagent with the standard creatinine solution (250 µmol·L⁻¹) in the volume ratio of 14:1, the dependence of the fluorescence intensity on the time was investigated (λₑₓ = 405 nm, λₑₘ = 480 nm). The change in NaOH concentration within the range of 0.75-1 mol·L⁻¹ does not significantly affect the kinetic course of the reaction. 2 mol·L⁻¹ was selected as the optimal concentration as it provides a slightly faster reaction between creatinine and DNBA (5000 counts/450 s). Interestingly, after an hour, the fluorescence intensity for all tested concentrations is equal to the fluorescence intensity recorded at the beginning of the chemical reaction, which means that the fluorescent product is likely to completely degrade during this time. The results are shown in Fig. 7.

In result of the optimisation experiments, the following concentrations of the components in the fluorimetric reagent were selected: DNBA (12.5 mmol·L⁻¹), NaOH (1 mol·L⁻¹), H₂O₂ (29 mmol·L⁻¹), 1,4-butanediol and water in a volume ratio of 0.8:1.

After optimisation of the fluorimetric reagent composition, the calibration characteristics and validation parameters of the method according to the invention were determined. For this purpose, a reagent with the optimal composition indicated above was prepared, as well as a series of creatinine standard solutions with concentrations ranging from 1-1000 µmοl·L⁻¹ After mixing the reagent with the creatinine standard solution in a volume ratio of 14:1, the fluorescence intensity of the creatinine-DNBA reaction product was measured after three different incubation times: 200 s, 300 s, 630 s (λₑₓ = 405 nm, λₑₘ = 480 nm). The calibration dependencies are shown in Fig. 8, and the basic validation parameters are given in Table 2.

**Table 2. Basic analytical parameters of the fluorimetric method of creatinine determination according to the invention for the reagent containing 12.5 mmol·L⁻¹ DNBA, 1 mol·L⁻¹ NaOH, 29 mmol·L⁻¹ H₂O₂, 1,4-butanediol and water in a 0.8:1 volume ratio. 14:1 volume ratio of reagent to sample. 200 µL sample volume. DL - limit of creatinine detection, QL - limit of creatinine quantification. The limits of detection and quantification of creatinine were calculated as the concentration corresponding to the mean of the measurement for the blank plus 3 or 10 standard deviations, resp.; n = 10.**

| **Incubation time** | **Equation of characteristics** | **R²** | **Linearity** [µmol·L⁻¹] | **DL** [µmol·L⁻¹] | **QL** [µmol·L⁻¹] | **Mean RSD** |
|---|---|---|---|---|---|---|
| 200 s | y = 11,8x + 429 | 0,994 | 6,8-750 | 2,3 | 6,8 | 3,86% |
| 300 s | y = 18,7x + 446 | 0,989 | 2,6-750 | 0,9 | 2,6 | 3,35% |
| 630 s | y = 23,0x + 406 | 0,981 | 0,9-500 | 0,3 | 0,9 | 2,90% |

The developed creatinine protocol shows linearity within the range 2.6-750 µmol·L⁻¹. The sensitivity of the method increases with the incubation time in the first phase (average 1-800 s), and then decreases due to the varying fluorescence intensity over time (Fig. 7), with an intense increase in fluorescence intensity following a 20 second incubation. This means that the optimal time for carrying out the measurement is on average in the range of up to about 800 s, but it is possible to conduct research in the entire range of fluorescence time (20-3600 s). The method according to the invention can be used to test biological samples because the physiological range of creatinine in these fluids is within or is greater than the linear range of the method, which allows for possible dilution of the fluids (blood serum 45-110 µmol·L⁻¹, urine 4-18 mmol·L⁻¹, dialysate 20-1000 µmol·L⁻¹).

### The influence of proteins on possibility of creatinine determination in water-organic environment

In order to check protein interference in the method of creatinine determination according to the invention, the dependence of the fluorescence intensity on the time (λₑₓ = 405 nm, λₑₘ = 480 nm) was recorded for the creatinine standard solution of the concentration of 250 µmol·L⁻¹ and for the same standard containing the addition of albumin (10-40 g·L⁻¹). The results of this experiment are shown in Fig. 9. It was observed that the presence of albumin starts to influence the fluorimetric signal after a certain time and not from the beginning of the reaction. Initially, i.e. up to 200 seconds of the reaction time, the fluorescence intensity is independent of the albumin concentration in the sample. This result suggests that it is possible to determine creatinine in the blood serum and without diluting the sample. It is worth noting that lower concentrations of albumin (10-20 g·L⁻¹) begin to reduce the signal only after about 300 seconds of the reaction between creatinine and DNBA. These are the concentrations of proteins lower than in the blood serum (approx. 40 g·L⁻¹), but in the case of dilution it is possible to extend the measurement time, which is advantageous due to the method sensitivity increasing with time (0-800 s).

In order to checl< whether the degree of albumin interference depends on the creatinine concentration, a series of creatinine standard solutions with the addition of albumin at a concentration of 40 g·L⁻¹ were prepared. The dependences of the fluorescence intensity (λₑₓ = 405 nm, λₑₘ = 480 nm) were recorded for the incubation time of 200 s. The obtained results are shown in Fig. 10. It was observed that the protein at the physiological concentration is not a significant interferent of the method according to the invention at creatinine concentrations below 200 µmol L⁻¹, because the calibration curve recorded with and without protein overlaps in this range. This means that irrespective of the creatinine concentration, it is possible to discriminate between the influence of proteins on the determination by measuring the fluorescence intensity after a maximum of 200 seconds for undiluted serum and for diluted serum after at least 300 seconds.

### Optimisation of the composition of the fluorimetric reagent based on an aqueous solvent

The optimisation of the reaction conditions began with the selection of the optimal concentration of H₂O₂ in a fluorimetric reagent in which DNBA was dissolved in an aqueous NaOH solution. A series of reagent solutions containing DNBA (12.5 mmol·L⁻¹), NaOH (1 mol·L⁻¹) and H₂O₂ in various concentrations ranging from 0-145 mmol·L⁻¹ were prepared. After mixing the reagent with the standard creatinine solution (250 µmol·L⁻¹) in the volume ratio of 14:1, the dependence of the fluorescence intensity on the time was investigated (λₑₓ = 405 nm, λₑₘ = 480 nm). Concentration of 29 mmol·L⁻¹ was selected as the optimal concentration of H₂O₂, analogous to the experiment with 1,4-butanediol. The results are shown in Fig. 11.

In order to optimise the concentration of DNBA in the fluorimetric reagent, a series of reagent solutions containing H₂O₂ (29 mmol·L⁻¹) and DNBA dissolved in NaOH solution in the DNBA/NaOH molar ratio equal to 2:3 was prepared, with the DNBA concentration fluctuating in the range of 0-50 mmol·L⁻¹. After mixing the reagent with the creatinine standard solution (250 µmol·L⁻¹) in a volume ratio of 14:1, the dependence of the fluorescence intensity on time was investigated (λₑₓ = 405 nm, λₑₘ = 480 nm). The highest fluorescence intensity was recorded for a DNBA concentration of 7.5 mmol·L⁻¹. The results are shown in Fig. 12.

The optimal concentration of NaOH in the fluorimetric reagent was determined after the preparation of a series of reagent solutions containing DNBA (7.5 mmol·L⁻¹), H₂O₂ (29 mmol·L⁻¹) and NaOH in various concentrations ranging from 0-2 mol·L⁻¹. After mixing the reagent with the standard creatinine solution (250 µmol·L⁻¹) in the volume ratio of 14:1, the dynamics of the analytical signal increase (λₑₓ = 405 nm, λₑₘ = 480 nm) was tested. It was observed that the use of NaOH in a concentration in the range of 0.75-1.5 mol·L⁻¹ gives similar results, but the concentration of 1 mol·L⁻¹ gives the greatest sensitivity. The results are shown in Fig. 13.

In effect of the optimisation experiments, the following concentrations of the components in the fluorimetric reagent not containing organic solvents were selected: DNBA (7.5 mmol·L⁻¹), NaOH (1 mol·L⁻¹), H₂O₂ (29 mmol·L⁻¹).

Calibration characteristics of the method were determined in accordance with the developed protocol. For this purpose, creatinine standard solutions with the concentration range of 1-1000 µmol·L⁻¹ were prepared and the fluorescence intensity was recorded after three reaction times: 200 s, 300 s, 630 s. The calibration curves for the fluorimetric creatinine determination method are shown in Fig. 14 (λₑₓ = 405 nm, λₑₘ = 480 nm). The analytical parameters are presented in Table 3.

It was observed that elimination of the organic solvent from the reaction medium causes a great loss of sensitivity of the method according to the invention and an increase in the limits of quantification and detection of creatinine. Wiling to determine serum creatinine without the use of an organic solvent, even a fivefold dilution may be at risk of obtaining a solution with a creatinine concentration lower than its limit of quantification. A possible solution to this problem may be to increase the width of the slots for the excitation and the emitted beams, but such an approach may reduce the selectivity of the method.

**Table 3. Basic analytical parameters of the fluorimetric creatinine determination method according to the invention for a reagent containing 7.5 mmol·L⁻¹ DNBA, 1 mol·L⁻¹ NaOH, 29 mmol·L⁻¹ H₂O₂. 14:1 volume ratio of reagent to sample. 200 µL sample volume. DL - creatinine detection limit, QL - limit of quantification of creatinine. The limits of detection and quantification of creatinine were calculated as the concentration corresponding to the mean measurement for the blank plus 3 or 10 standard deviations, resp.; n = 10.**

| **Incubation time** | **Equation of characteristics** | **R²** | **Linearity** [µmol·L⁻¹] | **DL** [µmol·L⁻¹] | **QL** [µmol·L⁻¹] | **Mean RSD** |
|---|---|---|---|---|---|---|
| 200 s | y = 4,4x + 588 | 0,998 | 25-1000 | 8,3 | 25 | 2,54% |
| 300 s | y = 7,5x + 585 | 0,996 | 10-1000 | 3,3 | 10 | 2,61% |
| 630 s | y = 8,4x + 543 | 0,995 | 8-1000 | 2,7 | 8,0 | 3,56% |

### The influence of proteins on the possibility of creatinine determination in water environment

In order to investigate the influence of proteins on the kinetics of fluorescence (λₑₓ = 405 nm, λₑₘ = 480 nm), a creatinine standard solution (250 µmol·L⁻¹) was prepared with different albumin content (10-40 g·L⁻¹) and the dependence of fluorescence intensity on time was recorded after mixing it with the reagent of optimal composition presented above. The results are shown in Fig. 15. It was observed that the influence of proteins is significant after a very short incubation time (approx. 110 s), which makes difficult the reliable determination of serum creatinine carried out according to the method of the invention using an aqueous reagent.

To investigate whether the observed relationship is present for other creatinine concentrations, a calibration curve was constructed without protein and in the presence of 40 g·L⁻¹ albumin in the standard. The time when the presence of proteins starts to lower the signal is short, and is approx. 100 seconds. For time in which the sensitivity of the method would be the highest due to the potentially high intensity of fluorescence (approx. 400 s), interferences from proteins prevent a reliable determination of creatinine. The results are shown in Fig. 16.

### The volume ratio of the reagent to the biological sample

Optimisation of the volume ratio of the reagent to a sample is needed due to the two opposing trends: increasing the sample volume increases the amount of analyte and lowering the limit of detection, and in turn reducing the sample volume, the degree of interference of other components of the sample decreases. Control measurements were carried out by mixing the water-organic reagent with the sample in volume ratios of 1:1, 14:1 and 299:1, and the concentrations of components in the obtained mixture were constant and amounted to 12.5 mmol·L⁻¹ DNBA, 1 mol·L⁻¹ NaOH, 29 mmol·L⁻¹ H₂O₂, and the volume ratio of 1,4-butanediol and water was 0.8:1. A series of creatinine standard solutions (250 µmol·L⁻¹) containing a number of potential interferents (glucose, uric acid, bilirubin, albumin, phosphates, urea) were prepared. After mixing the reagent with standard solutions in appropriate volume ratios, the dependence of fluorescence intensity on time was examined (λₑₓ = 405 nm, λₑₘ = 480 nm) and the comparative results are presented in Figs. 17 and 18. In the presence of interferents, the highest fluorescence intensity was observed for the systems in ratio of 14:1, and the 1:1 and 299:1 systems showed lower fluorescence intensity.

The interference of creatinine determination due to the presence of proteins (e.g. albumin) decreased with the increase in the ratio of the fluorescent reagent volume to the sample volume, because the amount of interferents in the tested mixture also decreased. For the 1:1 samples, the relative error due to the presence of proteins was as high as 100% (Fig. 19), and for the 14:1 samples it dropped to as little as 25% (Fig. 20). In turn, the detection limit increased with an increase in the V_{REAGENT}/V_{SAMPLE} ratio. Significant dilution of the sample at a ratio of 299:1 during the measurement increased the limit of detection for creatinine to a value of about 30 µmol·L⁻¹, which still allowed the assay to be performed, but indicated that further increasing the volume ratio would practically exclude the usefulness of such a protocol. On the other hand, the use of the V_{REAGENT}/V_{SAMPLE} = 1:1 protocol allowed to reduce the limit of creatinine detection significantly below 1 µmol·L⁻¹, (Table 4), but caused a significant (90%) extinction of the fluorescence of the product of creatinine reaction with anions of 3,5-dinitrobenzoic acid caused by the interfering proteins present in significant concentrations in the sample.

**Table 4. Basic analytical parameters of the fluorimetric method for determination of creatinine according to the invention for a reagent containing 25 mmol·L⁻¹ DNBA, 0.75 mol·L⁻¹ NaOH, 44 mmol·L⁻¹ H₂O₂, 1,4-butanediol and water in a 0.8:1 volume ratio. 1:1 volume ratio of reagent to sample. Sample volume 1500 µL. DL - limit of creatinine detection, QL - limit of creatinine quantification. The limits of detection and quantification of creatinine were calculated as the concentration corresponding to the mean measurement of the blank plus 3 or 10 standard deviations, resp.; n = 10.**

| **Incubation time** | **Equation of characteristics** | **R²** | **Linearity** [µmol·L⁻¹] | **DL** [µmol·L⁻¹] | **QL** [µmol·L⁻¹] | **Mean RSD** |
|---|---|---|---|---|---|---|
| 200 s | y = 44x + 204 | 0,996 | 1,4-250 | 0,86 | 2,5 | 4,91% |
| 300 s | y = 93x + 204 | 0,989 | 0,66-250 | 0,26 | 0,75 | 4,82% |
| 630 s | y = 114x + 223 | 0,971 | 0,52-250 | 0,38 | 1,3 | 4,67% |

Analytical measurements of the mixture of fluorimetric reagent and a sample in a volume ratio of 14:1 were considered to be the optimal protocol. When using such a protocol, the method of the invention has low detection and quantification limits, as well as high selectivity, because none of the potential interferents significantly influenced the recorded fluorimetric signal - the error caused by the presence of most interferents is not greater than ±7% for incubation times below 300 s.

### Comparison of the method according to the invention with the Jaffé method

The selectivity of the inventive fluorimetric creatinine determination method was compared to the Jaffé single point and two-point methods and to the photometric method based on o the creatinine reaction with DNBA by examining creatinine standard solutions containing admixtures of significant interferents present in biological samples (Fig. 21). As can be seen, the method according to the invention has the greatest selectivity for creatinine. Importantly, the error caused by the presence of bilirubin in the sample has been almost completely eliminated. In case of the Jaffé method, to get similar result it is necessary to make a two-point measurement, which is not preferred in analytical methods. Moreover, compared to other methods, the error caused by the presence of proteins is significantly reduced. In this form, the fluorimetric method for determining creatinine can be successfully applied to the determination of creatinine in blood serum, but also in urine and dialysate, where the problem of protein interference generally does not occur.

### Use of the method according to the invention in the measurement of synthetic sera

In order to verify the suitability of the method according to the invention for clinical trials, measurements were carried out on synthetic sera with normal (Cormay Serum HN) and pathological (Cormay Serum HP) content of creatinine and other components, as well as in their mixtures in dilutions ranging from 1 to 10 (Table 5).

The creatinine content of the samples was determined by the method of the invention using a fluorimetric reagent of an optimised composition: DNBA (12.5 mmol·L⁻¹), NaOH (1 mol·L⁻¹), H₂O₂ (29 mmol·L⁻¹), V_{BTD}/V_{H₂O} = 0.8:1, V_{REAGENT}/V_{SAMPLE} = 14:1. The cuvette was closed with a stopper and its contents was mixed. The results are shown in Fig. 22 and Table 5.

It has been observed that dilution of the serum sample prior to measurement is necessary to obtain reliable results. Creatinine concentrations determined in the undiluted serum sample significantly deviate from the nominal concentrations. The measurement time should depend on the degree of sample dilution - the more diluted the sample, the longer the incubation time should be used, for example: 200 s for a 2x dilution, 300 s for 5x, 630 sec for 10x. This trend is consistent with the results described above (Figs. 9, 10, 15, 16). For high sample dilution, the influence of proteins on the measurement is eliminated, but a long incubation time is advantageous due to the increased sensitivity of the method. On the other hand, for smaller protein dilutions, proteins reduce the analytical signal and therefore it is necessary to shorten the incubation time.

**Table 5. The content of creatinine and protein in the Cormay control sera and the results of determinations by the method according to the invention using the fluorimetric reagent: DNBA (12.5 mmol·L⁻¹), NaOH (1 mol·L⁻¹), H₂O₂ (29 mmol·L⁻¹), 1,4-butanediol and water in a volume ratio of 0.8:1. V_{REAGENT}/V_{SAMPLE} = 14:1. Measurements were carried out for various dilutions of sera, for incubation times of 200 s, 300 s, 630 s. Results within the concentration range declared by the manufacturer are marked in bold.**

| **Sample** | **Creatinine concentration** [µmol·L⁻¹] | **Protein content** [g·L⁻¹] | **Degree of sample dilution** | **Determination according to the method of the invention** | | |
|---|---|---|---|---|---|---|
| | | | | **200 s** [µmol·L⁻¹] | **300 s** [µmol·L⁻¹] | **630 s** [µmol·L⁻¹] |
| HN | 162 | 64,6 | 1x | 100 ± 2 | 57 ± 16 | 39 ± 1 |
| HN | 162 | 64,6 | 2x | **144 ± 13** | 118 ± 8 | 92 ± 4 |
| HN | 162 | 64,6 | 5x | 218 ± 4 | **170 ± 5** | 130 ± 1 |
| HN | 162 | 64,6 | 10x | 300 ± 13 | 230 ± 1 | **150 ± 5** |
| HP | 407 | 39,7 | 2x | **408 ± 16** | **348 ± 16** | 276 ± 10 |
| HP | 407 | 39,7 | 5x | 563 ± 25 | 475 ± 15 | **380 ± 16** |
| HP | 407 | 39,7 | 10× | 713 ± 7 | 560 ± 12 | **440 ± 17** |
| 1HN/1HP | 285 | 52,2 | 2x | **249 ± 9** | 222 ± 8 | 136 ± 25 |
| 1HN/1HP | 285 | 52,2 | 5x | **318 ± 29** | **285 ± 19** | 240 ± 8 |
| 1HN/1HP | 285 | 52,2 | 10x | 387 ± 25 | 342 ± 6 | **266 ± 3** |
| 3HN/1HP | 223 | 58,4 | 2x | **216 ± 0** | 179 ± 3 | 113 ± 17 |
| 3HN/1HP | 223 | 58,4 | 5x | 280 ± 3 | **241 ± 4** | 184 ± 2 |
| 3HN/1HP | 223 | 58,4 | 10x | 358 ± 5 | 291 ± 8 | **218 ± 9** |

### Use of the method according to the invention for the measurements of blood sera

An optimised protocol was used for the analysis of biological samples. Serum samples were selected for testing because they usually have a greater number of interferents in comparison with urine and dialysate samples which are usually easier to analyse. Samples were obtained from patients of the Independent Public Central Clinical Hospital. During the measurement carried out according to the method of invention, each one of the samples was diluted five times, and the reaction time was chosen to be 300 seconds. Detailed protocol for creatinine determination is provided in the working example. Comparative results were obtained using the enzymatic method at the Central Clinical Laboratory of the Medical University of Warsaw [Cobas, Creatinine plus ver.2, (2009)], and using the Jaffé protocols for one-point and two-point measurement modes.

A correlation was observed between the results obtained by the method according to the invention and the results obtained at the Central Clinical Laboratory using the enzymatic method. The comparison of the results with the determined 95% confidence interval is shown in Fig. 23. The linear regression equation of the obtained points is y = (0.94 ± 0.03) x + (3.66 ± 3.22) with the Prearson's coefficient r being 0.996. This result indicates a correlation between the two data sets. Moreover, the agreement between the results obtained by the fluorimetric method and the enzymatic method is shown in the Bland-Altman plot (Fig. 24), which is a graphic illustration of the agreement between the two measurement methods [D. Giavarina, Biochem. Medica. 25 (2015) 141-151]. All the points of the plot are within the agreement interval, i.e. inside the region between the standard deviation lines, which means that the enzymatic method and the method of the invention can be used interchangeably for the determination of creatinine in biological samples.

Moreover, in order to verify whether the obtained results differ significantly from the results obtained by the enzymatic method, two-tailed Student's t-test was performed for the confidence interval of 95% and 12 degrees of freedom. The determined value of the tₑₓₚ parameter is 1.499, while the critical (tabular) value of the t_{crit} parameter for this type of test is 2.179. This means that the creatinine concentrations determined by the method of the invention do not differ significantly from the creatinine concentrations determined by the enzymatic method, which is considered the most reliable routine method for creatinine detection.

During the analysis of the results obtained by the Jaffé method, two samples were rejected due to the very large standard deviation of the measurement series. The Student's t-test was used again to check the statistical differences in the results, but after the rejection of two samples, the number of degrees of freedom decreased to 10. Comparing the results obtained with the method according to the invention and the two-point Jaffé method, the tₑₓₚ parameter was 0.155, which is less than the critical t_{crit} value for this test of 2.228. It is also worth mentioning that when comparing the results obtained in the Clinical Laboratory by the enzymatic method with the one-point Jaffe method for a measurement time of 120 seconds, the determined value of the tₑₓₚ parameter is 2.964, i.e. it is higher than the critical value. This means that the concentrations determined by the one-point method of the invention are in better agreement with the results obtained with the enzymatic method in the Clinical Laboratory than the results obtained by the one-point Jaffé method.

### Cost analysis

The method for determining creatinine according to the invention shows exceptional selectivity, accuracy and precision when compared to the known fluorimetric methods. The results obtained with the method of the invention are comparable with standard measurement methods of other types, but in contrast, the method according to the invention is the only one that uses commercially available reagents. This is beneficial because the dangers and risks of their use, their impact on analysts' health, and recommended waste disposal methods are well known.

The comparison of the cost of performing 100 analyses (the cost of the reagents alone in terms of the total volume of the reagents and the sample, amounting to 3 mL), shows a similar price range of the optimised method according to the invention (approx. 30 PLN) and the Jaffé method (approx. 13 PLN). It should be noted, however, that 98% of the determination costs in the method according to the invention relates to the purchase cost of 1,4-butanediol, and replacing it with another solvent (e.g. propylene glycol) can significantly reduce the total cost of the reagents below 13 PLN, i.e. below the cost level of the Jaffé method. For comparison, a ready-made commercial enzyme kit allowing to perform 100 determinations costs almost € 450 (about 2,000 PLN) [https://www.abcam.com/creatinine-assay-kit-ab65340.html, 14/06/2019]. The cost difference between the method according to the invention and the enzyme method is significant.

### Conclusions

The fluorimetric method according to the invention allows for a reliable and precise determination of creatinine concentration in clinically significant biological samples. The results obtained by the method according to the invention are consistent with the results obtained in the Clinical Laboratory using a routine enzymatic method. It is a one-point method, requiring relatively short incubation times (200-630 s), which eliminates the inconvenience of enzymatic methods. The determination range of creatinine is in the range of 0.5-1000 µmol·L⁻¹, for incubation times of 20-3600 s, preferably 3-750 µmol·L⁻¹ for 300 s. The method according to the invention allows the measurement of creatinine content in blood serum samples, urine or dialysate after appropriate dilution, and the dialysate often does not need to be diluted.

The speed and simplicity of measurements, as well as the use of non-toxic components of the fluorimetric reagent, make it possible to create measuring systems ready for use at any time and place, in accordance with the concept of *point of care testing.* The unit measurement price is competitive with other routinely used methods of creatinine determination in biological samples.

The invention is described below in the working examples illustrating the implementation of the method according to the invention using the reagent according to the invention, by means of their use for the determination of creatinine in clinically significant biological samples.

**Example 1. Fluorimetric determination of creatinine in blood serum:** Two component solutions were prepared. Solution 1: aqueous-organic solution of 3,5-dinitrobenzoic acid (25 mmol·L⁻¹) and hydrogen peroxide (58 mmol·L⁻¹) in a mixture of 1,4-butanediol and water in a 9:1 volume ratio. Solution 2: NaOH aqueous solution (2 mol·L⁻¹). Into the fluorimetric cuvette there were successively introduced: 1.4 mL of Solution 1, and then 1.4 mL of Solution 2, thus obtaining the fluorimetric reagent of the following composition: DNBA (12.5 mmol·L⁻¹), NaOH (1 mol·L⁻¹), H₂O₂ (29 mmol·L⁻¹), 1,4-butanediol and water in a 0.8:1 volume ratio. The reaction was initiated by introducing a 0.2 mL of a sample of 5-fold diluted blood serum into a fluorimetric cuvette, resulting in a mixture of 14:1 volume ratio of the reagent and sample. The content of the cuvette was mixed for 30 s, and then the registration of changes in the fluorescence intensity of the creatinine-DNBA reaction product was started. The value recorded after 300 s of incubation (λₑₓ = 405 nm, λₑₘ = 480 nm) was compared with a previously prepared calibration curve to determine the creatinine concentration in the sample, and the reading was compared with the reference data. The obtained result was within the 95% confidence interval of the reference method (Hitachi Roche Cobas 6000) - enzyme protocol.

**Example 2. Fluorimetric determination of creatinine in urine.** The procedure was as in Example 1. The reaction was initiated by loading into a fluorimetric cuvette a 0.2 mL sample of 50-fold diluted urine to obtain a 14:1 volume ratio of reagent and sample. The obtained result was within the 95% confidence interval of the reference method (Hitachi Roche Cobas 6000) - Jaffé protocol.

**Example 3. Fluorimetric determination of creatinine in a dialysate.** The procedure was as in Example 1. The reaction was initiated by loading into a fluorimetric cuvette a 0.2 mL sample of 2-fold diluted dialysate to obtain a 14:1 v/v mixture of reagent and sample. The obtained result was within the 95% confidence interval of the reference method (Hitachi Roche Cobas 6000) - Jaffé protocol.

## Claims

1. A fluorimetric analytical method for the determination of creatinine in clinically significant biological samples, in which creatinine is reacted with 3,5-dinitrobenzoic acid in a strongly all<aline aqueous-organic medium to form a fluorophore product, **characterised in that** a clinically significant biological sample is mixed directly with a multi-component fluorimetric reagent containing 3,5-dinitrobenzoate anions, an organic solvent, water, a base maintaining alkalinity of the reagent above pH = 12, and an addition of hydrogen peroxide, whereas detection and/or quantification of the obtained product of creatinine reaction with 3,5-dinitrobenzoate anions is carried out using the fluorimetric method, by taking a measurement of radiation having a wavelength of 450-500 nm, preferably 480 nm, emitted under the influence of an excitation radiation beam of a wavelength of 380-420 nm, preferably 405 nm, at a constant incubation time after the start of the reaction and determining creatinine concentration on the basis of separately prepared calibration curve that links creatinine concentration in the biological sample with the fluorimetric response characteristic for the given wavelength and incubation time.

2. The method according to claim 1, **characterised in that** as a component containing 3,5-dinitrobenzoate anions, a solution of 3,5-dinitrobenzoic acid, a 3,5-dinitrobenzoate salt, preferably sodium 3,5-dinitrobenzoate or 3,5-dinirobenzoic acid ester, preferably methyl 3,5-dinitrobenzoate is used, the solution comprising an organic solvent, a mixture of organic solvents, water, or a mixture thereof.

3. The method according to claim 2, **characterised in that** as the organic solvent, a solvent which is highly miscible with water, preferably a monohydric alcohol or a polyhydric alcohol is used, wherein as the monohydric alcohol, methanol, ethanol, propanol or butanol is used and as the polyhydric alcohol, 1,4-butanediol, 1,2-propanediol or ethylene glycol is used, wherein 1,4-butanediol is the most preferred.

4. The method according to claim 1, **characterised in that** as the base-containing component, a base solution having a concentration greater than 0.05 mol·L⁻¹, wherein the solution comprises water, an organic solvent or a mixture of organic solvents, or a mixture thereof, wherein, as the base, a metal hydroxide, ammonium hydroxide or an organic base that does not significantly interfere with the fluorescence of the product of creatinine reaction with a 3,5-dinitrobenzoate anion, preferably an all<ali metal hydroxide (LiOH, NaOH, KOH, RbOH, CsOH), ammonium hydroxide, tetramethylammonium hydroxide, tetraethylammonium hydroxide, tetrabutylammonium hydroxide, or mixtures thereof, most preferably LiOH, NaOH or KOH, is used.

5. The method according to claim 1, **characterised in that** as the component containing hydrogen peroxide, an aqueous or aqueous-organic hydrogen peroxide solution is used.

6. The method according to claim 1, **characterised in that** the fluorimetric reagent is made of components including 3,5-dinitrobenzoic acid, 1,4-butanediol, water, NaOH and hydrogen peroxide, the components being mixed in any order and combination prior to adding the biological sample of clinical significance, wherein, stable component solutions are primarily prepared, preferably a component solution containing 3,5-dinitrobenzoic acid, 1,4-butanediol, water and hydrogen peroxide, and a component solution containing water and NaOH, which component solutions are mixed immediately before adding the biological sample of clinical significance.

7. The method according to claim 1, **characterised in that** for samples containing creatinine in concentrations lower than or equal to 750 µmol·L⁻¹, the reagent is used in which:
- the concentration of 3,5-dinitrobenzoate ions ranges from 0.05 mmol·L⁻¹ up to saturation, preferably being 12.5 mmol·L⁻¹,
- the base concentration is 0.05-2 mol-L¹, preferably 1 mol·L⁻¹,
- the concentration of hydrogen peroxide is 1.45-145 mmol·L⁻¹, preferably 29 mmol·L⁻¹,
- the mutual ratio of solvents ranges from completely aqueous to aqueous-organic containing 0.3% water, and the optimal ratio of organic solvent to water in the water-organic mixture is from 0.8:1,
while the samples containing creatinine in concentrations greater than 750 µmol·L⁻¹ are diluted to a concentration lower than or equal to 750 µmol·L⁻¹.

8. The method according to claim 1, **characterised in that** the volume ratio of the fluorimetric reagent to the clinically significant biological sample is from 1:1 to 299:1, preferably 14:1.

9. The method according to claim 1, **characterised in that** the measurement of the fluorescence intensity is performed in the range of the observed linear dependence of the fluorescence intensity on the creatinine concentration, i.e. after the time of 20-3600 s, preferably 300 s.

10. The method according to claim 1, **characterised in that** the creatinine determination range is within the range of 0.5-1000 µmol·L⁻¹ for the incubation time of 20-3600 s, preferably 3-750 µmol·L⁻¹ for the incubation time of 300 s.

11. The method according to claim 1, **characterised in that** it is selective for creatinine in the presence of sugars, proteins and bilirubin.

12. The method according to claim 1, **characterised in that** clinically significant biological sample is
- a blood serum at a 2-10-fold dilution, preferably at 5-fold dilution;
- a urine at a 10-400-fold dilution, preferably at 50-fold dilution;
- a dialysate at a 1-5-fold dilution, preferably at 2-fold dilution.

13. A multi-component fluorimetric reagent, being alkaline and containing 3,5-dinitrobenzoate anions in an aqueous-organic environment, **characterised in that** it additionally contains hydrogen peroxide and 1,4-butanediol, is suitable for use in the analytical fluorimetric method for determination of creatinine in clinically significant biological samples, and after direct mixing with a clinically significant biological sample containing creatinine, selectively produces a fluorophore in the reaction between creatinine and a 3,5-dinitrobenzoate anion, which fluorophore under the influence of an excitation radiation beam of a wavelength of 380-420 nm, preferably 405 nm, emits radiation of a wavelength of 450-500 nm, preferably 480 nm, wherein
- a concentration of 3,5-dinitrobenzoate ions is from 0.05 mmol·L⁻¹ up to saturation, preferably 12.5 mmol·L⁻¹;
- concentration of a base is 0.05-2 mol·L⁻¹, preferably 1 mol·L⁻¹;
- concentration of hydrogen peroxide is 1.45-145 mmol·L⁻¹, preferably 29 mmol·L⁻¹;
- the mutual ratio of the solvents, water and 1,4-butanediol, ranges from completely organic to fully aqueous, and the optimal ratio of organic solvent to water in the aqueous-organic mixture is from 0.8:1,
which fluorimetric reagent is stable at room temperature for over 100 hours after its preparation and can be used for the selective determination of creatinine at concentrations ranging from 3-750 µmol·L⁻¹ in the presence of glucose, proteins and bilirubin, for an incubation time of 20-3600 s, preferably 300 s.

## Patentansprüche

1. Fluorimetrisches Analyseverfahren zur Bestimmung von Kreatinin in klinisch signifikanten biologischen Proben, bei dem Kreatinin mit 3,5-Dinitrobenzoesäure in einem stark alkalischen wässrig-organischen Medium umgesetzt wird, um ein Fluorophorprodukt zu bilden, **dadurch gekennzeichnet, dass** eine klinisch signifikante biologische Probe direkt mit einem mehrkomponentigen fluorimetrischen Reagens vermischt wird, das 3,5-Dinitrobenzoat-Anionen, ein organisches Lösungsmittel, Wasser, eine Base, die pH des Reagens oberhalb 12 hält, und ein Zusatz von Wasserstoffperoxid enthält, wobei die Detektion und/oder Quantifizierung des erhaltenen Produkts der Reaktion von Kreatinin mit 3,5-Dinitrobenzoat-Anionen unter Verwendung des fluorimetrischen Verfahrens durchgeführt wird, indem eine Strahlenemission mit einer Wellenlänge von 450-500 nm, vorzugsweise 480 nm, gemessen wird, die unter dem Einfluss eines Anregungsstrahlenbündels mit einer Wellenlänge von 380-420 nm, vorzugsweise 405 nm, bei einer konstanten Inkubationszeit nach dem Beginn der Reaktion emittiert wird, und die Konzentration von Kreatinin auf der Grundlage einer separat erstellten Kalibrierungskurve, die die Konzentration von Kreatinin in der biologischen Probe mit der für die gegebene Wellenlänge und Inkubationszeit charakteristischen fluorimetrischen Antwort verknüpft, bestimmt wird.

2. Verfahren nach Anspruch 1, **dadurch gekennzeichnet, dass** als die 3,5-Dinitrobenzoat-Anionen enthaltende Komponente, eine Lösung von 3,5-Dinitrobenzoesäure, einem 3,5-Dinitrobenzoatsalz, vorzugsweise Natrium-3,5-Dinitrobenzoat oder 3,5-Dinirobenzoesäureester, vorzugsweise Methyl-3,5-Dinitrobenzoat, verwendet wird, wobei die Lösung ein organisches Lösungsmittel, eine Mischung organischer Lösungsmittel, Wasser oder eine Mischung davon enthält.

3. Verfahren nach Anspruch 2, **dadurch gekennzeichnet, dass** als organisches Lösungsmittel ein mit Wasser gut mischbares Lösungsmittel, vorzugsweise ein einwertiger Alkohol oder ein mehrwertiger Alkohol, verwendet wird, wobei als einwertiger Alkohol Methanol, Ethanol, Propanol oder Butanol und als mehrwertiger Alkohol 1,4-Butandiol, 1,2-Propandiol oder Ethylenglykol, verwendet wird, wobei 1,4-Butandiol am meisten bevorzugt ist.

4. Verfahren nach Anspruch 1, **dadurch gekennzeichnet, dass** als die basenhaltige Komponente eine Basenlösung mit einer Konzentration von mehr als 0,05 mol·L⁻¹ verwendet wird, wobei die Lösung Wasser, ein organisches Lösungsmittel oder eine Mischung von organischen Lösungsmitteln oder eine Mischung davon, enthält, wobei als die Base ein Metallhydroxid, Ammoniumhydroxid oder eine organische Base verwendet wird, die die Fluoreszenz des Produkts der Umsetzung von Kreatinin mit einem 3,5-Dinitrobenzoat-Anion nicht wesentlich stört, vorzugsweise ein Alkalimetallhydroxid (LiOH, NaOH, KOH, RbOH, CsOH), Ammoniumhydroxid, Tetramethylammoniumhydroxid, Tetraethylammoniumhydroxid, Tetrabutylammoniumhydroxid oder Mischungen davon, am bevorzugtesten LiOH, NaOH oder KOH verwendet wird.

5. Verfahren nach Anspruch 1, **dadurch gekennzeichnet, dass** als wasserstoffperoxidhaltige Komponente eine wässrige oder wässrig-organische Wasserstoffperoxidlösung verwendet wird.

6. Verfahren nach Anspruch 1, **dadurch gekennzeichnet, dass** das fluorimetrische Reagens aus Komponenten hergestellt wird, die 3,5-Dinitrobenzoesäure, 1,4-Butandiol, Wasser, NaOH und Wasserstoffperoxid umfassen, wobei die Komponenten vor Zugabe der klinisch signifikanten biologischen Probe in beliebiger Reihenfolge und Kombination vermischt werden, wobei zunächst stabile Komponentenlösungen hergestellt werden, vorzugsweise eine Komponentenlösung, die 3,5-Dinitrobenzoesäure, 1,4-Butandiol, Wasser und Wasserstoffperoxid enthält, und eine Komponentenlösung, die Wasser und NaOH enthält, wobei die Komponentenlösungen unmittelbar vor Zugabe der klinisch signifikanten biologischen Probe vermischt werden.

7. Verfahren nach Anspruch 1, **dadurch gekennzeichnet, dass** für Proben, die Kreatinin in Konzentrationen kleiner oder gleich 750 µmol·L⁻¹ enthalten, das Reagens verwendet wird, bei dem:
- die Konzentration der 3,5-Dinitrobenzoat-lonen von 0,05 mmol·L⁻¹ bis zur Sättigung reicht, vorzugsweise 12,5 mmol·L⁻¹ beträgt,
- die Basenkonzentration von 0,05 bis 2 mol·L¹, vorzugsweise 1 mol·L⁻¹ beträgt,
- die Konzentration des Wasserstoffperoxids von 1,45 bis 145 mmol·L⁻¹, vorzugsweise 29 mmol·L⁻¹ beträgt,
- das Verhältnis der Lösungsmittel zueinander von vollständig wässrig bis wässrig-organisch mit 0,3% Wasser reicht und das optimale Verhältnis von organischem Lösungsmittel zu Wasser in der wässrig-organischen Mischung 0,8:1 beträgt,
während die Proben, die Kreatinin in Konzentrationen größer als 750 µmol·L⁻¹ enthalten, auf eine Konzentration kleiner oder gleich 750 µmol·L⁻¹ verdünnt werden.

8. Verfahren nach Anspruch 1, **dadurch gekennzeichnet, dass** das Volumenverhältnis des fluorimetrischen Reagens zur klinisch signifikanten biologischen Probe von 1:1 bis 299:1, vorzugsweise 14:1, beträgt.

9. Verfahren nach Anspruch 1, **dadurch gekennzeichnet, dass** die Messung der Fluoreszenzintensität im Bereich der beobachteten linearen Abhängigkeit der Fluoreszenzintensität von der Kreatininkonzentration, d.h. nach einer Zeit von 20 bis 3600 s, vorzugsweise 300 s, durchgeführt wird.

10. Verfahren nach Anspruch 1, **dadurch gekennzeichnet, dass** der Bestimmungsbereich von Kreatinin im Bereich von 0,5 bis 1000 µmol·L⁻¹ bei einer Inkubationszeit von 20 bis 3600 s, vorzugsweise von 3 bis 750 µmol·L⁻¹ bei einer Inkubationszeit von 300 s liegt.

11. Verfahren nach Anspruch 1, **dadurch gekennzeichnet, dass** es in Gegenwart von Zuckern, Proteinen und Bilirubin selektiv für Kreatinin ist.

12. Verfahren nach Anspruch 1, **dadurch gekennzeichnet, dass** die klinisch signifikante biologische Probe
- ein Blutserum in 2-10-facher Verdünnung, vorzugsweise in 5-facher Verdünnung, ist;
- ein Urin in 10-400-facher Verdünnung, vorzugsweise in 50-facher Verdünnung, ist;
- ein Dialysat in 1-5-facher Verdünnung, vorzugsweise in 2-facher Verdünnung, ist.

13. Ein mehrkomponentiges fluorimetrisches Reagens, das alkalisch ist und 3,5-Dinitrobenzoat-Anionen in einer wässrig-organischen Umgebung enthält, **dadurch gekennzeichnet, dass** es zusätzlich Wasserstoffperoxid und 1,4-Butandiol enthält, für Verwendung in der analytischen fluorimetrischen Methode zur Bestimmung von Kreatinin in klinisch signifikanten biologischen Proben, geeignet ist, und nach direktem Mischen mit einer klinisch signifikanten biologischen Probe, die Kreatinin enthält, selektiv einen Fluorophor in der Reaktion zwischen Kreatinin und einem 3,5-Dinitrobenzoat-Anion bildet, der unter dem Einfluss eines Anregungsstrahlbündels von einer Wellenlänge von 380 bis 420 nm, vorzugsweise 405 nm, Strahlung von einer Wellenlänge von 450 bis 500 nm, vorzugsweise 480 nm, emittiert, worin
- Konzentration von 3,5-Dinitrobenzoat-lonen von 0,05 mmol·L⁻¹ bis zur Sättigung, vorzugsweise 12,5 mmol·L⁻¹, beträgt;
- Konzentration einer Base von 0,05 bis 2 mol·L¹, vorzugsweise 1 mol·L⁻¹, beträgt;
- Konzentration von Wasserstoffperoxid von 1,45 bis 145 mmol·L⁻¹, vorzugsweise 29 mmol·L⁻¹, beträgt;
- das Verhältnis der Lösungsmittel (Wasser und 1,4-Butandiol) zueinander von vollständig organisch bis vollständig wässrig reicht, und das optimale Verhältnis von organischem Lösungsmittel zu Wasser in der wässrig-organischen Mischung 0,8:1 beträgt,
welches fluorimetrisches Reagenz nach seiner Herstellung bei Raumtemperatur über 100 Stunden stabil ist und zur selektiven Bestimmung von Kreatinin in Konzentrationen von 3 bis 750 µmol·L⁻¹ in Gegenwart von Glucose, Proteinen und Bilirubin bei einer Inkubationszeit von 20 bis 3600 s, vorzugsweise 300 s, verwendet werden kann.

## Revendications

1. Procédé d'analyse fluorimétrique pour la détermination de la créatinine dans des échantillons biologiques cliniquement significatifs, dans lequel la créatinine est mise à réagir avec de l'acide 3,5-dinitrobenzoïque dans un milieu aqueux-organique fortement alcalin pour former un produit fluorophore, **caractérisé en ce que** l'échantillon biologique cliniquement significatif est mélangé directement avec un réactif fluorimétrique à plusieurs composants contenant des anions 3,5-dinitrobenzoate, un solvant organique, de l'eau, une base maintenant l'alcalinité du réactif au-dessus de pH = 12, et un ajout de peroxyde d'hydrogène, tandis que la détection et/ou la quantification du produit obtenu de la réaction de la créatinine avec les anions 3,5-dinitrobenzoate est réalisée à l'aide du procédé fluorimétrique, en effectuant une mesure de rayonnement ayant une longueur d'onde de 450 à 500 nm, de préférence 480 nm, émis sous l'influence d'un faisceau de rayonnement d'excitation d'une longueur d'onde de 380 à 420 nm, de préférence 405 nm, à un temps d'incubation constant après le début de la réaction et en évaluant la concentration de créatinine sur la base d'une courbe d'étalonnage préparée séparément qui relie la concentration de créatinine dans l'échantillon biologique à la réponse fluorimétrique pour la longueur d'onde et le temps d'incubation donnés.

2. Procédé selon la revendication 1, **caractérisé en ce que** comme le composant contenant des anions 3,5-dinitrobenzoate on utilise une solution d'acide 3,5-dinitrobenzoïque, un sel de 3,5-dinitrobenzoate, de préférence le 3,5-dinitrobenzoate de sodium ou l'ester d'acide 3,5-dinirobenzoïque, de préférence le 3,5-dinitrobenzoate de méthyle, une solution comprenant un solvant organique, un mélange de solvants organiques, de l'eau ou un mélange de ceux-ci.

3. Procédé selon la revendication 2, **caractérisé en ce que** comme le solvant organique on utilise un solvant hautement miscible à l'eau, de préférence un alcool monohydrique ou un alcool polyhydrique, l'alcool monohydrique étant le méthanol, l'éthanol, le propanol ou le butanol et l'alcool polyhydrique étant le 1,4-butanediol, le 1,2-propanediol ou l'éthylène glycol, le 1,4-butanediol étant le plus préféré.

4. Procédé selon la revendication 1, **caractérisé en ce que** comme le composant contenant une base on utilise une solution basique ayant une concentration supérieure à 0,05 mol·L⁻¹, la solution comprenant de l'eau, un solvant organique ou un mélange de solvants organiques, ou un mélange de ceux-ci, la base étant un hydroxyde métallique, un hydroxyde d'ammonium ou une base organique qui n'interfère pas de manière significative avec la fluorescence du produit de réaction de la créatinine avec l'anion 3,5-dinitrobenzoate, de préférence un hydroxyde de métal alcalin (LiOH, NaOH, KOH, RbOH, CsOH), un hydroxyde d'ammonium, un hydroxyde de tétraméthylammonium, un hydroxyde de tétraéthylammonium, un hydroxyde de tétrabutylammonium, ou leurs mélanges, de préférence LiOH, NaOH ou KOH.

5. Procédé selon la revendication 1, **caractérisé en ce que** comme le composant contenant du peroxyde d'hydrogène on utilise une solution aqueuse ou aqueuse-organique du peroxyde d'hydrogène.

6. Procédé selon la revendication 1, **caractérisé en ce que** le réactif fluorimétrique est constitué de composants comprenant de l'acide 3,5-dinitrobenzoïque, du 1,4-butanediol, de l'eau, du NaOH et du peroxyde d'hydrogène, les composants étant mélangés dans n'importe quel ordre et combinaison avant d'ajouter l'échantillon biologique cliniquement significatif, dans lequel des solutions de composants stables sont préparées en premier lieu, de préférence une solution de composants contenant de l'acide 3,5-dinitrobenzoïque, du 1,4-butanediol, de l'eau et du peroxyde d'hydrogène, et une solution de composants contenant de l'eau et du NaOH, lesquelles solutions de ces composants sont mélangées immédiatement avant d'ajouter l'échantillon biologique cliniquement significatif.

7. Procédé selon la revendication 1, **caractérisé en ce que** pour les échantillons contenant de la créatinine dans des concentrations inférieures ou égales à 750 µmol·L⁻¹, on utilise le réactif dans lequel :
- la concentration en ions 3,5-dinitrobenzoate varie de 0,05 mmol·L⁻¹ jusqu'à la saturation, de préférence est de 12,5 mmol·L⁻¹,
- la concentration en base est de 0,05 à 2 mol·L¹, de préférence est de 1 mol·L⁻¹,
- la concentration en peroxyde d'hydrogène est de 1,45 à 145 mmol·L⁻¹, de préférence est de 29 mmol·L⁻¹,
- le rapport mutuel des solvants varie de complètement aqueux à aqueux-organique contenant 0,3% d'eau, et le rapport optimal du solvant organique à l'eau dans le mélange eau-organique est de 0,8:1,
tandis que les échantillons contenant de la créatinine dans des concentrations supérieures à 750 µmol·L⁻¹ sont dilués à une concentration inférieure ou égale à 750 µmol·L⁻¹.

8. Procédé selon la revendication 1, **caractérisé en ce que** le rapport volumique du réactif fluorimétrique à l'échantillon biologique cliniquement significatif est de 1:1 à 299:1, de préférence est de14:1.

9. Procédé selon la revendication 1, **caractérisé en ce que** la mesure de l'intensité de fluorescence est effectuée dans la plage de la dépendance linéaire observée de l'intensité de fluorescence sur la concentration de la créatinine, c'est-à-dire après un temps de 20 à 3600 s, de préférence 300 s.

10. Procédé selon la revendication 1, **caractérisé en ce que** la plage de détermination de la créatinine est comprise entre 0,5 et 1000 µmol·L⁻¹ pour une durée d'incubation de 20 à 3600 s, de préférence de 3 à 750 µmol·L⁻¹ pour une durée d'incubation de 300 s.

11. Procédé selon la revendication 1, **caractérisé en ce qu'**il est sélectif pour la créatinine en présence des sucres, des protéines et de la bilirubine.

12. Procédé selon la revendication 1, **caractérisé en ce que** l'échantillon biologique cliniquement significatif est
- un sérum sanguin dilué de 2 à 10 fois, de préférence dilué de 5 fois ;
- une urine diluée de 10 à 400 fois, de préférence diluée de 50 fois ;
- un dialysat dilué de 1 à 5 fois, de préférence dilué de 2 fois.

13. Réactif fluorimétrique à plusieurs composants, qui est alcalin et contient des anions 3,5-dinitrobenzoate dans un environnement aqueux-organique, **caractérisé en ce qu'**il contient en outre du peroxyde d'hydrogène et du 1,4-butanediol, est adapté pour destiné à être utilisé dans la méthode fluorimétrique analytique pour la détermination de la créatinine dans des échantillons biologiques cliniquement significatifs, après mélange direct avec un échantillon biologique cliniquement significatif contenant de la créatinine, produit sélectivement un fluorophore dans la réaction entre la créatinine et l'anion 3,5-dinitrobenzoate, lequel fluorophore sous l'influence d'un faisceau de rayonnement d'excitation d'une longueur d'onde de 380 à 420 nm, de préférence de 405 nm, émet un rayonnement d'une longueur d'onde de 450 à 500 nm, de préférence de 480 nm, où
- la concentration d'ions 3,5-dinitrobenzoate est de 0,05 mmol·L⁻¹ jusqu'à saturation, de préférence de 12,5 mmol·L⁻¹ ;
- la concentration d'une base est de 0,05-2 mol·L⁻¹, de préférence de 1 mol·L⁻¹ ;
- la concentration du peroxyde d'hydrogène est de 1,45 à 145 mmol·L⁻¹, de préférence de 29 mmol·L⁻¹ ;
- le rapport mutuel des solvants (d'eau et du 1,4-butanediol) varie de complètement organique à complètement aqueux, et le rapport optimal du solvant organique à l'eau dans le mélange aqueux-organique est de 0,8:1,
lequel réactif fluorimétrique est stable à température ambiante pendant plus de 100 heures après sa préparation et peut être utilisé pour la détermination sélective de la créatinine à des concentrations allant de 3 à 750 µmol·L⁻¹ en présence du glucose, des protéines et de la bilirubine, pour une durée d'incubation allant de 20 à 3600 s, de préférence de 300 s.
